# EUROPEAN PATENT SPECIFICATION

(11) **EP 3 196 133 B1**
(45) Date of publication and mention of the grant of the patent: **14.03.2018**
(21) Application number: 16151947.5
(22) Date of filing: 19.01.2016
(51) Int. Cl.: B65B 9/10, A61F 13/551

(54) **IMPROVED WRAPPING DEVICE AND METHOD**
VERBESSERTE VERPACKUNGSVORRICHTUNG UND VERFAHREN
DISPOSITIF D'EMBALLAGE AMÉLIORÉ ET PROCÉDÉ

(43) Date of publication of application: 26.07.2017
(73) Proprietor: Ontex BVBA, 9255 Buggenhout (BE)
(72) Inventor: Heege, Thomas, 56727 Mayen (DE); Heege, Rudolf, 56727 Mayen (DE)
(74) Representative: Brantsandpatents bvba

(56) References cited:
- EP-A1- 1 477 406
- EP-B1- 0 108 095
- GB-A- 827 087
- US-A1- 2005 070 865

## Description

### Technical field

The invention pertains to the technical field of manufacturing packaging for elongate products, and more specifically packaging for tampons or similar products, whereby the packaging is manufactured separately from the product, tampons in some cases. The invention comprises an improved apparatus for the manufacture of said packaging and an improved method of manufacturing said packaging.

### Background

Tampons are well known in the art and are used for feminine hygiene. Also, many tampon manufacturing methods and apparatuses have been disclosed in prior art documents. Tampons, and in particular digital tampons, can be covered by a packaging which preferably closely fits the tampon.

Many of the specific prior art methods disclosed are directed towards the immediate packaging of the tampon, whereby the packaging is directly applied to the tampon and manipulated while being on or around the tampon. Document EP1477406A1, for instance, discloses such a packaging method, wherein the tampon is inserted inside a tubular folding spindle of constant cross section; a sheet of packing material is then wrapped about the folding spindle and stabilized longitudinally to form a tubular wrapping; the folding spindle and the tubular wrapping, together with the tampon, are then parted; and the two ends of the tubular wrapping are closed transversely about the relative tampon. Heat sealing of tubular packagings are also known from for instance EP 1,227,979 A1, EP 0,970,888A1 or US 5,533,323 A for cellophaning cigarette packets.

Other apparatuses are disclosed in US 4,845,922 B whereby the tampons are loaded intermittently into a long tube which is continuously shaped from a wrapping material that is provided and sealed such that the formed tube closely fits the encased tampons. The tube is then cut before and after each encased tampon whereby the cuts are closed. However, this process is very slow and does not provide very accurate packagings.

However, the present invention relates to another technique, wherein the packaging is manufactured at least partly, and preferably completely, separate from the tampon, i.e. the packaging is essentially manufactured in a suitable form before it is combined with the tampon. Such a method allows for an optimized total production process of packaged tampons. Indeed, if the tampons are packaged directly, the process of forming the tampons and the process of forming the packages need to be performed inline, whereby the total production process speed needs to be optimized taking into account both processes. The present invention allows the production process for the tampon and the production process for the packaging to be at least partly performed in parallel, such that one is capable of optimizing each process separately, thereby also capable of increasing total process speeds. Furthermore, by forming the packaging on the tampon itself, the manufacturing of the packaging would likely influence the tampon. Commonly used techniques such as heat-sealing of the packaging could damage the tampon or have other undesirable effects.

One possible method and apparatus for manufacturing tampon packagings has been developed by Ruggli. Hereby, a strip of thermoplastic film is supplied at the inlet of a wheel to a holding element and then fixed by a staff or rod shaped clamping means. Once the strip is fixed in position, the wheel starts to move around the rotational axis of the wheel. At the first stop (after a rotation of about 45°), the strip is wrapped around the holding means and both of its ends that meet, are heat sealed together in a first heat sealing station, thusly forming a "tube". At a second stop (after another rotation of about 45°), the distal end of the tube (facing away from the center of the wheel) is heat sealed and closed in two distinct heat sealing stations, the third and second ones. The cellophane hull (now having a cylindrical body with one open end) is then released at the outlet of the "cellophane wheel" to another wheel where it will be later combined with the tampon.

Another such technique is disclosed in EP 0,108,095 B where the wrapping material is sucked up by a winding mandrel and wound around said mandrel by rotating the mandrel, after which the transversal ends of the wrapping material are joined to each other by heat-sealing, thus creating a packaging tube. Again, the proposed concept does not offer a method or apparatus which can operate at the speeds desired to match that of other manufacturing processes for manufacturing the tampons, and this would lead to the speed of the entire process being dictated by the slow process suggested in EP 0,108,095 B.

The problem with the above described packaging methods comes down to the fact that the production speed is not high enough to keep up with present-day production speeds of tampon manufacturing apparatuses. As a result, more than one of the prior art tampon packaging manufacturing apparatuses needs to be combined with a tampon manufacturing apparatus in order to increase total production speed. It should be clear that such a solution to increase total production speed is not ideal.

There remains a need in the art for an improved apparatus for manufacturing tampon packaging. The inventors have found a method and apparatus for this process by which the package production speed of the Ruggli method described above can be substantially improved by up to a factor of 2 and even more. It is to be noted that the Ruggli method and apparatuses are commonly used in this field.

### Summary of the invention

The present invention provides a method and apparatus for wrapping strips of film around a holding means, more specifically wrapping said strips of film to manufacture elongate packagings, even more specifically tampon packagings.

In a first aspect, the invention provides a method for wrapping a strip of film around a holding means, specifically the holding means of a tampon packaging manufacturing apparatus, comprising the steps of:
a. providing a strip, preferably a quadrilateral strip and more preferably a rectangular strip, preferably of heat-sealable film;
b. providing an elongate holding means having a longitudinal axis parallel to the strip;
c. displacing the strip along a displacement axis essentially perpendicular to said longitudinal axis towards the holding means and/or displacing the holding means along a displacement axis essentially perpendicular to said longitudinal axis towards the strip;
d. pressing the strip against the holding means between a first end of the strip and a second end of the strip;
e. while the strip is pressed against the holding means, wrapping the first end of the strip around the holding means in a first rotational direction and wrapping the second end of the strip around the holding means in a second rotational direction opposite to the first rotational direction;
f. optionally immobilizing the wrapped strip of film with respect to the holding means; and
g. optionally sealing, preferably heat-sealing, an overlap of the first and the second end of the strip.

In a preferred embodiment of the method, the wrapping of the first end of the strip around the holding means and wrapping of the second end of the strip around the holding means is executed so that at least part of the first end overlaps with at least part of the second end of the strip, characterized in that the at least part of the second end of the strip is wrapped between the holding means and the at least part of the first end of the strip.

In a preferred embodiment of the method, the strip is pressed against the holding means between a first end of the strip and a second end of the strip in a first pressing area and a second pressing area, whereby the first pressing area continuously moves over the holding means during the wrapping of the first end of the strip and the second pressing area continuously moves over the holding means during the wrapping of the second end of the strip.

In a preferred embodiment of the method, in step c of the aforementioned method the strip is displaced along the displacement axis essentially perpendicular to the longitudinal axis and towards the holding means, preferably wherein the holding means remains stationary, during the step of displacing the strip, pressing the strip and wrapping the strip, and preferably during the step of sealing the strip and/or wherein in step c the holding means are displaced along a displacement axis essentially perpendicular to said longitudinal axis and towards the strip, preferably wherein the housing remains stationary, during the step of displacing the strip, pressing the strip and wrapping the strip, and preferably during the step of sealing the strip.

In a second aspect, the invention provides an apparatus for wrapping a strip of film around an elongate holding means to manufacture an elongate, preferably essentially cylindrical, packaging, and especially suitable as tampon packaging.

In said second aspect, the apparatus comprises a support and a housing which is displaceably mounted on said support. The apparatus further comprises a first set of embracing means and a second set of embracing means, the first set of embracing means being mounted rotatably onto the housing around a first pivot axis and the second set of embracing means being mounted rotatably onto the housing around a second pivot axis which is essentially parallel to the first pivot axis. The housing is is displaceably mounted on the support and is reversibly movable with respect to said support along a displacement axis essentially towards the holding means for pushing the strip of film towards the holding means by at least one of said embracing means, said displacement axis essentially perpendicular to said first and second pivot axes, thereby defining a pinching direction essentially perpendicular to said displacement axis and to said first and second pivot axes; and/or the holding means is reversibly movable along a displacement axis essentially towards the housing for pushing the strip of film towards the embracing means, said displacement axis essentially perpendicular to said first and second pivot axes, thereby defining a pinching direction essentially perpendicular to said displacement axis and to said first and second pivot axes. Each set of embracing means comprises at least one embracing means, whereby the embracing means comprises: a pressing means for pressing the strip of film between the embracing means and the holding means; and a wrapping means for wrapping the strip of film around the holding means. The pressing means of the at least one embracing means of the first set of embracing means is displaceable along at least the pinching direction with respect to the pressing means of the at least one embracing means of the second set of embracing means. The wrapping means of the embracing means of the first set of embracing means is configured to execute a wrapping movement for wrapping the strip of film around the holding means in a first rotational direction while the embracing means are being displaced and the pressing means are pressed against the holding means. The wrapping means of the embracing means of the second set of embracing means is configured to execute a wrapping movement for wrapping the strip of film around the holding means in a second rotational direction opposite to the first rotational direction, while the embracing means are being displaced and the pressing means are pressed against the holding means.

In a preferred embodiment, the pressing means of at least one embracing means of the first set of embracing means is displaceable along at least the pinching direction with respect to the housing and the at least one embracing means is configured so that a displacement with respect to the housing of said pressing means of the at least one embracing means of the first set of embracing means along at least the pinching direction, actuates the wrapping movement of the wrapping means of the at least one embracing means of the first set of embracing means and/or the pressing means of at least one embracing means of the second set of embracing means is displaceable along at least the pinching direction with respect to the housing and whereby the at least one embracing means is configured so that a displacement with respect to the housing of said pressing means of the at least one embracing means of the second set of embracing means along at least the pinching direction, actuates the wrapping movement of the wrapping means of the at least one embracing means of the second set of embracing means. Preferably the pressing means of all the embracing means of the first set of embracing means are displaceable along at least the pinching direction with respect to the housing and whereby all of the embracing means are configured so that a displacement with respect to the housing of said pressing means of one or more of the embracing means of the first set of embracing means along at least the pinching direction, actuates the wrapping movement of the wrapping means of said one or more of the embracing means of the first set of embracing means. Preferably the pressing means of all the embracing means of the second set of embracing means are displaceable along at least the pinching direction with respect to the housing and whereby all of the embracing means are configured so that a displacement with respect to the housing of said pressing means of one or more of the embracing means of the second set of embracing means along at least the pinching direction, actuates the wrapping movement of the wrapping means of said one or more of the embracing means of the second set of embracing means.

In a preferred embodiment, the pressing means of at least one embracing means of the first set of embracing means is adapted to continuously move over the holding means in the second rotational direction while the at least one embracing means of the first set of embracing means is being displaced, preferably wherein the pressing means of all the embracing means of the first set of embracing means are adapted to continuously move over the holding means in the second rotational direction while the embracing means of the first set of embracing means are being displaced and/or wherein the pressing means of at least one embracing means of the second set of embracing means is adapted to continuously move over the holding means in the first rotational direction while the at least one embracing means of the second set of embracing means is being displaced, preferably wherein the pressing means of all the embracing means of the second set of embracing means are adapted to continuously move over the holding means in the first rotational direction while the embracing means of the second set of embracing means are being displaced.

In a preferred embodiment, the first set of embracing means is capable of wrapping a first end of the strip around the holding means in the first rotational direction and the second set of embracing means is capable of wrapping a second end of the strip opposite to the first end of the strip, around the holding means in the second rotational direction, whereby at least part of the wrapped first end of the strip and at least part of the wrapped second end of the strip are superimposed. Herein the first set of embracing means and the second set of embracing means are configured with respect to each other so that the superimposed part of the first end of the strip is positioned between the holding means and the superimposed part of the second strip.

In a preferred embodiment, the apparatus comprises: a film providing means for providing the film; a cutting means for cutting the film, thereby forming the strip of film and providing said strip of film between the holding means and the first and second set of embracing means; and optionally a supporting means for supporting the strip of film between the holding means and the first and second set of embracing means.

In a preferred embodiment, the apparatus comprises a conveying means which comprises one or more of said holding means and is configured to provide the holding means in a stop-go process to a stationary position which is essentially distanced from the first and the second set of embracing means along the displacement axis, preferably whereby the conveying means is a conveying wheel capable of rotating with a multitude of said holding means equidistantially disposed along the edge of said conveying wheel.

In a preferred embodiment, the pressing means of at least one embracing means of the first and/or the second set of embracing means comprises one or more rollers, preferably the pressing means of all the embracing means of the first and the second set of embracing means comprise one or more rollers. The one or more rollers are adapted to continuously move over the holding means while the housing is being displaced along the displacement axis and while the one or more rollers press against the holding means.

In a preferred embodiment, the wrapping means of at least one embracing means of the first and/or the second set of embracing means comprises one or more extensions, preferably the wrapping means of all of the embracing means of the first and the second set of embracing means comprises one or more extensions. The one or more extensions are adapted to execute the wrapping movement for wrapping the strip of film around the holding means while the housing is being displaced along the displacement axis and while the one or more pressing means press against the holding means.

In a preferred embodiment, at least one and preferably all of the embracing means of the first and/or the second set are connected to the housing by one or more springs and whereby the springs are configured to actuate the pressing of the pressing means against the holding means.

In a preferred embodiment, the wrapping means are configured to wrap the strip around the holding means so that a first end of the strip and an opposite, second end of the strip of film are superimposed, and whereby the apparatus comprises one or more fixating means adapted to clamp the superimposed ends of the wrapped strip of film and/or seal, preferably heat-seal, said superimposed ends of the wrapped strip of film, preferably whereby the apparatus comprises one of said fixating means for each of the holding means.

In a preferred embodiment, the apparatus comprises a rotatable cam and one or more returning means, whereby the housing is mounted onto the support such that the rotatable cam is adapted to actuate a displacement of the housing essentially towards the holding means, and whereby the one or more returning means are adapted to actuate a displacement of the housing essentially away from the holding means.

In a third aspect, the invention provides a tampon packaging manufacturing apparatus comprising a holding means, an apparatus for wrapping a strip of film around an elongate holding means as described in this document, and one or more sealing means for sealing a strip of film wrapped around the holding means to manufacture an elongate, preferably essentially cylindrical, tampon packaging.

### Description of figures

**FIG. 1** shows an isometric view of a possible housing according to the invention.
**FIG. 2** shows an outer view of the apparatus according to the invention.
**FIG. 3** shows a frontal view of a possible embodiment of the embracing means in non-operative mode.
**FIG. 4** shows an exposed (without housing) side view of a possible embodiment of the embracing means in non-operative mode, with a holding means in front of the embracing means.
**FIG. 5** shows an exposed (without housing) side view of a possible embodiment of the embracing means in non-operative mode, with a holding means in front of the embracing means.
**FIG. 6** shows a side view of a possible embodiment of the apparatus, with a holding means in front of the embracing means, and a fixating means behind the holding means.
**FIG. 7** shows a side view of the cutting means.

### Detailed description of the invention

In a first aspect, the invention provides a method for wrapping a strip of film around a holding means, specifically the holding means of a tampon packaging manufacturing apparatus, comprising the steps of:
a. providing a strip, preferably a quadrilateral strip and more preferably a rectangular strip, preferably of heat-sealable film;
b. providing an elongate holding means having a longitudinal axis parallel to the strip;
c. displacing the strip along a displacement axis essentially perpendicular to said longitudinal axis towards the holding means and/or displacing the holding means along a displacement axis essentially perpendicular to said longitudinal axis towards the strip;
d. pressing the strip against the holding means between a first end of the strip and a second end of the strip;
e. while the strip is pressed against the holding means, wrapping the first end of the strip around the holding means in a first rotational direction and wrapping the second end of the strip around the holding means in a second rotational direction opposite to the first rotational direction;
f. optionally immobilizing the wrapped strip of film with respect to the holding means; and
g. optionally sealing, preferably heat-sealing, an overlap of the first and the second end of the strip.

The execution of step c can be performed in 3 distinct ways. In the first option, the holding means remain stationary while the strip is displaced towards the holding means. The advantages of such a setup is that the holding means can easily be implemented by a practical conveying system, such as a wheel, that offers holding means to the apparatus and that the holding means does not need to execute complex motions, and that the holding means cannot unduly influence the apparatus (for instance by slight variations in the movement of the holding means, push the apparatus out of its place).

The second option, wherein the strip remains stationary and the holding means moves towards the strip, has amongst its advantages that the apparatus executing the method can be more easily constructed.

Lastly, it is also possible to combine the abovementioned options, and have both the holding means and the embracing means move towards the strip of film.

The method of the invention is particularly suitable for wrapping a strip of film around a holding means in a tampon packaging manufacturing apparatus, as it strongly preferred that the strip of film is not directly applied to a tampon and thereupon shaped and sealed (heat-sealed usually) as this could damage the product. The same reasoning applies to several other products that can be so packaged. This method furthermore provides several advantages over the methods used in the general prior art, as well as over the methods specifically directed towards tampon packaging manufacturing. As mentioned, the method offers a significant increase in speed over the widely-used Ruggli method and apparatus. For instance, the design of a separate structure that executes the application (displacement and wrapping) of the strip around the holding means allows the applicant to use a one-step process, instead of the Ruggli method, where providing the strip and providing the holding means have to be adjusted to each other very carefully, as the strip is provided in the path of the conveyance direction of the holding means. Furthermore, the strip is first applied generally against the holding means, and at a later step and station wrapped around the holding means, and at a still later step and station heat-sealed. This requires three separate stations and separate steps and thusly results in a lower production speed. Especially the step of providing the strip is complicated in the Ruggli method, as on the one hand the conveyance means is intended to move at high speeds, but still has to stop for a long enough time for the strip to be placed in the path of the holding means and applied thereto. The applicant provides with a method where all these steps are combined in a single step.

In a preferred embodiment of the method, the wrapping of the first end of the strip around the holding means and wrapping of the second end of the strip around the holding means is executed so that at least part of the first end overlaps with at least part of the second end of the strip, characterized in that the at least part of the second end of the strip is wrapped between the holding means and the at least part of the first end of the strip.

By ensuring that the method always wraps the strip of film around the holding means in the same way, with the first end above the second end of the strip, this could make the process of clamping and/or sealing the overlapping ends easier. Furthermore, if this is not clearly regulated, the ends could be folded incorrectly, and could for instance be folded over themselves or bent double (superimpose themselves).

In a preferred embodiment of the method, the strip is pressed against the holding means between a first end of the strip and a second end of the strip in a first pressing area and a second pressing area, whereby the first pressing area continuously moves over the holding means during the wrapping of the first end of the strip and the second pressing area continuously moves over the holding means during the wrapping of the second end of the strip.

By executing this step so that the strip is pressed against the holding means in varying pressed areas or pressing areas, it is ensured that the strips fits tightly around the holding means and that no discontinuities or unwanted overlaps are present in the wrapped strip of film.

In a preferred embodiment of the method, in step c the strip is displaced along the displacement axis essentially perpendicular to the longitudinal axis towards the holding means, preferably wherein the holding means remains stationary, during the step of displacing the strip, pressing the strip and wrapping the strip, and preferably during the step of sealing the strip and/or wherein in step c the holding means are displaced along a displacement axis essentially perpendicular to said longitudinal axis towards the strip, preferably wherein the housing remains stationary, during the step of displacing the strip, pressing the strip and wrapping the strip, and preferably during the step of sealing the strip.

As before, this is dependent on the execution of step c, wherein either the strip can be moved towards the holding means, the holding means can be moved towards the strip, or both. In the first option, the holding means remains stationary while the strip is displaced to the holding means. In the second option, the holding means is moved towards the stationary strip, and in the third option, both the strip and the holding means move towards each other.

By holding the holding means stationary during the abovementioned steps, the process of wrapping the strip can be executed in a single, or at least several simultaneous, steps. Furthermore, it is preferred to have as few as possible directly interacting moving elements as this complicates both process and apparatus capable for said process. By holding the holding means stationary, the wrapping can be executed more easily by a wrapping sub-apparatus. Therefore, this is the most preferred embodiment, without however dismissing the other possibilities.

In a preferred embodiment of the method, the holding means moves along a conveyance path, in a conveyance direction, whereby the strip is provided such that it does not intersect the conveyance path of the holding means, preferably so that the strip is provided essentially parallel to the conveyance path, or the local conveyance direction of the provided holding means.

By providing the strip so that it does not intersect the further (or previous) conveyance path of the holding means, it is possible to use a conveying means with several holding means that can be provided at high speed. In the Ruggli method, the strip is provided between subsequent holding means and therefore, can only be provided when the holding means are stationary. In the method proposed by the applicant, the strip of film can be provided in a wider time range.

In said second aspect, the apparatus comprises a support and a housing which is displaceably mounted on said support. The apparatus further comprises a first set of embracing means and a second set of embracing means, the first set of embracing means being mounted rotatably onto the housing around a first pivot axis and the second set of embracing means being mounted rotatably onto the housing around a second pivot axis which is essentially parallel to the first pivot axis. The housing is is displaceably mounted on the support and is reversibly movable with respect to said support along a displacement axis essentially towards the holding means for pushing the strip of film towards the holding means by at least one of said embracing means, said displacement axis essentially perpendicular to said first and second pivot axes, thereby defining a pinching direction essentially perpendicular to said displacement axis and to said first and second pivot axes; and/or the holding means is reversibly movable along a displacement axis essentially towards the housing for pushing the strip of film towards the embracing means, said displacement axis essentially perpendicular to said first and second pivot axes, thereby defining a pinching direction essentially perpendicular to said displacement axis and to said first and second pivot axes. Each set of embracing means comprises at least one embracing means, whereby the embracing means comprises: a pressing means for pressing the strip of film between the embracing means and the holding means; and a wrapping means for wrapping the strip of film around the holding means. The pressing means of the at least one embracing means of the first set of embracing means is displaceable along at least the pinching direction with respect to the pressing means of the at least one embracing means of the second set of embracing means. The wrapping means of the embracing means of the first set of embracing means is configured to execute a wrapping movement for wrapping the strip of film around the holding means in a first rotational direction while the embracing means are being displaced and the pressing means are pressed against the holding means. The wrapping means of the embracing means of the second set of embracing means is configured to execute a wrapping movement for wrapping the strip of film around the holding means in a second rotational direction opposite to the first rotational direction, while the embracing means are being displaced and the pressing means are pressed against the holding means.

Many of the previously described advantages of the method of the invention hold true for the apparatus described in this document as well. It is ideal to be used in at least part of the manufacturing process for tampon packaging. As opposed to many of the known devices, the suggested apparatus does not apply the packaging directly to the tampon with the aforementioned advantages. Furthermore, the apparatus is adapted to execute an application step and a wrapping step in one fluid, fast motion instead of the several subprocesses and substations needed in other methods and systems. In at least one embodiment, the apparatus as described allows the embracing means to push a strip of film towards the stationary holding means, thereby pressing the strip against the holding means and wrapping the strip around the holding means in a single fluid movement. By not having the holding means actively participate in the wrapping process, the influence of the process on the holding means is minimized, thereby not disturbing possible further processes that need accurate positioning of said holding means. The strip is firmly pressed at at least one pressing area against the holding means, preferably at two pressing areas, one for the pressing means of each embracing means. More preferably said pressing area (or pressing areas) is a narrow zone that extends between the extremal ends of the cylindrical holding means, thereby ensuring a proper positioning and (temporary) fastening of the strip of film against the holding means, and thereby ensuring a sufficient wrapping operation following the pressing operation. The Ruggli apparatus has the holding means perform most of the wrapping operation, as it is moved towards the strip of film that is presented to the holding means. After this, the strip is fastened to the holding means and wrapped further by the following movement of the holding means. The applicant notes that this necessitates the presence of multiple stations to execute the entire wrapping process, and furthermore noticed that having the holding means move during the wrapping process, in fact slows down the process. The applicant notes that by having a single station that executes the providing of the strip and the application of the strip in one process, the entire system can execute the process for subsequently provided holding means at a higher speed, and thereby produce the packagings (or intermediate products for packagings) faster than other systems. The combination of both pressing and wrapping the strip is achieved by having at least two sets of embracing means, a first set designed to wrap the strip around the holding means in a first rotational direction, a second set to wrap the strip around the holding means in a second rotation direction opposite to the first rotational direction. By wrapping in both rotational directions, the effect of wrapping the entire strip around the holding means is achieved faster than by wrapping the strip in only one direction (arguably twice as fast). However, the embracing means also press the strip against the holding means, and it is by the combination of the pressing means of the embracing means pressing the strip against the holding means, and the displacement of the embracing means of the set of embracing means that the wrapping means of the embracing means executes a wrapping movement.

It is to be noted that the sets of embracing means comprise one or more embracing means, preferably there are three embracing means in each set of the embracing means. However, a different number of embracing means in each set is possible, as well as different numbers dependent on which set of embracing means is referred to. For instance, 2, 4, 5, 6, 7, 8, 9, 10, 12, 15, 20 or more embracing means can be comprised in each set.

Preferably, this wrapping movement follows the curved surface of the holding means (the mantle of the cylindrical holding means) and thereby ensures that the strip is wrapped correctly around the holding means. This can for instance be achieved by using the pressing area where the pressing means contacts the holding means as an input force with the pivot axis of the embracing means as fulcrum around which the embracing means, and thereby the wrapping means can be pivoted and is able to fold the strip around the holding means.

Note that the pinching direction in certain embodiment is not necessarily a straight line, but can also be curved, and preferably is a curved axis that follows at least part of the curve of the mantle of the holding means.

In a preferred embodiment, the pressing means of at least one embracing means of the first set of embracing means is displaceable along at least the pinching direction with respect to the housing and the at least one embracing means is configured so that a displacement with respect to the housing of said pressing means of the at least one embracing means of the first set of embracing means along at least the pinching direction, actuates the wrapping movement of the wrapping means of the at least one embracing means of the first set of embracing means and/or the pressing means of at least one embracing means of the second set of embracing means is displaceable along at least the pinching direction with respect to the housing and whereby the at least one embracing means is configured so that a displacement with respect to the housing of said pressing means of the at least one embracing means of the second set of embracing means along at least the pinching direction, actuates the wrapping movement of the wrapping means of the at least one embracing means of the second set of embracing means. Preferably the pressing means of all the embracing means of the first set of embracing means are displaceable along at least the pinching direction with respect to the housing and whereby all of the embracing means are configured so that a displacement with respect to the housing of said pressing means of one or more of the embracing means of the first set of embracing means along at least the pinching direction, actuates the wrapping movement of the wrapping means of said one or more of the embracing means of the first set of embracing means. Preferably the pressing means of all the embracing means of the second set of embracing means are displaceable along at least the pinching direction with respect to the housing and whereby all of the embracing means are configured so that a displacement with respect to the housing of said pressing means of one or more of the embracing means of the second set of embracing means along at least the pinching direction, actuates the wrapping movement of the wrapping means of said one or more of the embracing means of the second set of embracing means.

As said, in some embodiments the pressing area (of one or more embracing means) is adapted to move or be movable across the surface of the holding means during the wrapping movement. In certain embodiments which will be expanded upon later, this will in fact (co-)actuate the wrapping movement itself, as the pressing means will function as a moving point of contact which exerts an input force on the lever created by the embracing means around its pivot axis. This allows the wrapping means to follow the shape of the holding means more accurately during the wrapping movement, thereby ensuring optimal wrapping and not allowing the strip to avoid the wrapping means, for instance by slipping between the holding means and the wrapping means. Furthermore, it is preferable that the pressing area moves across the surface of the holding means during the wrapping movement, as this would more tightly and securely wrap the strip of film around the holding means. The pressing means is furthermore adapted to press against the holding means due to a force being exerted on the embracing means that tries to return the embracing means to their original position, for instance by the use of springs. These springs can be attached to the housing and to a distal end of the embracing means (distal with respect to the pressing means and thus having the pivot axis of the embracing means between the pressing means and said distal end), whereby the springs exert a force on the embracing means directed away from the holding means, and thereby causing the pressing means to be forced against the holding means, following its shape as it moves along.

In a preferred embodiment, the pressing means of at least one embracing means of the first set of embracing means is adapted to continuously move over the holding means in the second rotational direction while the at least one embracing means of the first set of embracing means is being displaced and/or the pressing means of at least one embracing means of the second set of embracing means is adapted to continuously move over the holding means in the first rotational direction while the at least one embracing means of the second set of embracing means is being displaced. Preferably the pressing means of all the embracing means of the first set of embracing means are adapted to continuously move over the holding means in the second rotational direction while the embracing means of the first set of embracing means are being displaced. Preferably the pressing means of all the embracing means of the second set of embracing means are adapted to continuously move over the holding means in the first rotational direction while the embracing means of the second set of embracing means are being displaced.

By having the pressing means continuously move over the holding means, it is further ensured that the strip is applied firmly and tightly to the holding means, thus creating more uniform packagings. The pressing means can be adapted so that they move over part of the surface of the holding means for each wrapping movement that is fully executed. This part of the surface can for instance be one twentieth, one fifteenth, one tenth, one eight, one sixth, one quarter, one third, one half, or the entire surface of the holding means. However, it is also possible that the pressing means are less extensive. In this case, it is easier to say that the pressing means move over a certain part of the circumference of the holding means, again, the same possibilities can be proposed as for the part of the circumference that is moved over by the holding means.

In a preferred embodiment, the first set of embracing means is capable of wrapping a first end of the strip around the holding means in the first rotational direction and the second set of embracing means is capable of wrapping a second end of the strip opposite to the first end of the strip, around the holding means in the second rotational direction, whereby at least part of the wrapped first end of the strip and at least part of the wrapped second end of the strip are superimposed. Herein the first set of embracing means and the second set of embracing means are configured with respect to each other so that the superimposed part of the first end of the strip is positioned between the holding means and the superimposed part of the second strip.

In order for the strip to be sealed after the wrapping movement, it is preferable that there is an overlap of at least parts of the extremal first and second ends of the strip. These ends can then be sealed together, for instance via heat-sealing. It is however possible that in certain embodiments, the overlapping of the first and the second end of the strip is not necessary and that an additional connecting strip is added that is sealed to the first and the second end, thereby connecting these ends, or that other connectors are used to connect the ends and seal these together.

The sealing operation can for instance be executed via more specific forms of heat-sealing such as ultrasonic welding, hot meld adhesive, induction welding and others. All of these processes allow fast sealing of the two ends without requiring elaborate machinery. More preferably, the devices necessary to perform the sealing operation are incorporated into the apparatus for wrapping the strip around the holding means, even more preferably so that the action of bonding the ends of the strip to each other is performed essentially simultaneously with wrapping the strip around the holding means, or directly subsequent to the wrapping. This would be ideal since, as the wrapping process may be concluded, the embracing means and essentially most of the apparatus for wrapping the strip around the holding means will need to retract somehow away from the holding means. This way, the sealing process can be implemented by the sealing devices without slowing the process down.

The control of which end is located 'beneath the other end (meaning which end is between the other end and the holding means) is especially important as this avoids possible interactions of the ends during the wrapping process. If the process were so that the relative position of the ends is random, then the ends could meet during the wrapping process and instead of neatly folding one end beneath the other, the ends could push up against each other and steer away (radially) from the holding means. This would result in a defective intermediate packaging product and is therefore to be avoided. The applicant proposes in the invention that the sets of embracing means are adapted so that it is always the first end of the strip that lies beneath the second end. This is for instance achieved by positioning one of the sets of the embracing means closer with respect to the other set of embracing means, to where the holding means is provided. In preferred embodiments, both sets of embracing means undergo the same displacement towards the holding means, which will allow the closer set of embracing means to perform the wrapping movement of said closer set earlier with respect to the wrapping movement of the other set of embracing means. However, this is merely one possibility that is offered to achieve the desired effect.

In a preferred embodiment, the apparatus comprises: a film providing means for providing the film; a cutting means for cutting the film, thereby forming the strip of film and providing said strip of film between the holding means and the first and second set of embracing means; and optionally a supporting means for supporting the strip of film between the holding means and the first and second set of embracing means.

As mentioned before, the apparatus as described in this document is particularly suited for use in a system that produces elongate (tampon) packagings from strips of film. Therefore, it is preferable that the apparatus is adapted so that a strip of film is provided to the apparatus for application to the holding means. This is most easily implemented as a film providing means and a cutting means, the first of which intermittently provides a length of film (wherein the length of the film is the preferably slightly larger than the circumference of the holding means). The cutting means is adapted to cut the length of film when it is provided into a strip of film, thereby allowing the apparatus to apply the strip of film to the holding means. More preferably, a supporting means is provided so as to hold the strip of film in the correct position for easier application to the holding means, and more importantly to avoid the strip of film to slip away from the apparatus. The film providing means and the cutting means are modified to cyclically perform their operations in sync with each other. The film providing means starts providing the film, once the desired length is provided, the film providing means stops providing the film and the cutting means cuts the strip of film as soon as possible. The strip of film can then come to rest upon the supporting means for a short time before the embracing means applies the strip to the holding means. Preferably, the time between each operation is reduced as much as possible to speed up the entire process and to avoid potential undesired effects (such as the strip starting to curl up, the strip falling off the supporting means and others).

In a preferred embodiment, the apparatus comprises a conveying means which comprises one or more of said holding means and is configured to provide the holding means in a stop-go process to a stationary position which is essentially distanced from the first and the second set of embracing means along the displacement axis, preferably whereby the conveying means is a conveying wheel capable of rotating with a multitude of said holding means equidistantially disposed along the edge of said conveying wheel.

The addition of a conveying means for providing holding means in a stop-go process in a stationary position to the embracing means, allows the apparatus to work at higher speeds as multiple holding means preferably are present. As soon as the strip of film is wrapped (and sealed), the holding means holding the wrapped strip of film is taken away and a new holding means is provided to which the same process is applied again. The speed and frequency of the conveying means is to be carefully matched to the speeds at which the embracing means (and possibly the film providing means and cutting means) operate so as to achieve an optimized process flow. Preferably, the holding means are attached to the conveying means towards the edge of the conveying means and whereby the longitudinal axis of the holding means is essentially perpendicular to the local movement of the stop-go process in which the holding means are conveyed. More specifically, in case of a conveying wheel, the longitudinal axis of the holding means is essentially parallel to the rotational axis of the conveying wheel, and the holding means are located equidistantially along the edge of the conveying wheel. However, in some possible embodiments, the holding means are attached to the conveying means or conveying wheel so that the longitudinal axis of the holding means is extending radially from the conveying means or conveying wheel.

In a preferred embodiment, the pressing means of at least one embracing means of the first and/or the second set of embracing means comprises one or more rollers, preferably the pressing means of all the embracing means of the first and the second set of embracing means comprise one or more rollers. The one or more rollers are adapted to continuously move over the holding means while the housing is being displaced along the displacement axis and while the one or more rollers press against the holding means.

The advantages of this embodiment have been described earlier in this document, rollers being the preferred embodiment of the pressing means has the advantage that these can easily both press the strip against the holding means and smooth out the strip while moving continuously over the holding means. Preferably, each embracing means comprises one roller, resulting in one or more rollers in each set of embracing means.

In a preferred embodiment, the wrapping means of at least one embracing means of the first and/or the second set of embracing means comprises one or more extensions, preferably the wrapping means of all of the embracing means of the first and the second set of embracing means comprises one or more extensions. The one or more extensions are adapted to execute the wrapping movement for wrapping the strip of film around the holding means while the housing is being displaced along the displacement axis and while the one or more pressing means press against the holding means. Even more preferably, the extensions are flexible in the plane that is perpendicular to the longitudinal axis of the holding means. The extensions can therefore wrap the strip around the holding means by pushing the strip against the holding means. If the extensions were not flexible, this could lead to the following setup and its negative consequences: in the first the extensions would be adapted so that they would not or barely touch the holding means to avoid breaking the extensions, and this could lead to the strip not being wrapped tight enough around the holding means or the strip even slightly curling up around the holding means, having air pockets, being folded double and other deficiencies. Furthermore, the strip could escape from between the extensions and the holding means, thereby not completing the wrapping process and leading to a poorly made product. In a preferred embodiment, each embracing means in every set of embracing means comprises an extension. These extensions are preferably placed at a distal end of the embracing means with respect to the pressing means so that the pressing movement actuates the wrapping movement by rotating the embracing means around its pivot axis.

In a preferred embodiment, at least one and preferably all of the embracing means of the first and/or the second set are connected to the housing by one or more springs, whereby the springs are configured to actuate the pressing of the pressing means against the holding means. More specific embodiments and advantages of this setup have been discussed in this document and will furthermore be expanded upon in the examples. When the sets of embracing means, and thereby the embracing means, are displaced towards the holding means, the springs will be remain in their original state wherein the embracing means are in a certain disposition or orientation with respect to the springs, whereby the longitudinal axis of the spring intersects with the pivot axis of the embracing means, resulting in a zero torque exerted by the embracing means. When the embracing means contact the holding means, the pressing means are pressed against the holding means and are no longer displaced along with the embracing means. In some embodiments, the pressing means remain stationary during the further displacement of the embracing means, however in most embodiments the pressing means move over the surface of the holding means. In both cases however, the pressing means do not follow the displacement of the embracing means of which it is part, as said. In fact, both cases result in a rotation of the embracing means around its pivot axis, so that the longitudinal axis of the spring no longer intersects the pivot axis of the embracing means, thereby causing a torque which produces a force pressing the pressing means against the holding means.

In a preferred embodiment, the wrapping means are configured to wrap the strip around the holding means so that a first end of the strip and an opposite, second end of the strip of film are superimposed, and whereby the apparatus comprises one or more fixating means adapted to clamp the superimposed ends of the wrapped strip of film and/or seal, preferably heat-seal, said superimposed ends of the wrapped strip of film, preferably whereby the apparatus comprises one of said fixating means for each of the holding means.

As mentioned before with other additions, the advantages of incorporating such fixating means in the apparatus would allow a more streamlined production process with the apparatus. Practically, a fixating means will clamp the superimposed ends of the wrapped strip and seal these ends together (possible ways to perform said sealing this have been discussed earlier) and one can argue that in order to seal the ends, these would be thereby also clamped. The fixating means is preferably adapted to act in concert with the embracing means so that when these retract or start to retract, the fixating means instantaneously clamp and/or seal the superimposed ends of the strip. This can be performed by pressing the fixating means against the superimposed ends, thereby trapping the superimposed ends between the fixating means and the holding means. The fixating means could comprise a sealing element that performs a sealing operation on the fixated superimposed ends.

In a preferred embodiment, the apparatus comprises a rotatable cam and one or more returning means whereby the housing is mounted onto the support such that the rotatable cam is adapted to actuate a displacement of the housing essentially towards the holding means, and whereby the one or more returning means are adapted to actuate a displacement of the housing essentially away from the holding means. Preferably said returning means are one or more springs which are attached to the housing at one end of the springs and to the support at the opposite end of the springs. When the cams actuate a displacement of the housing with respect to the support, the springs will exert a force on the housing, pulling it back into place once the cam allows the original displacement to be reversed. In some embodiments, the cam can also be used to actuate the cutting means to cut the strip of film, as the frequencies of pushing the housing towards the holding means and the cutting of the strip of film would be equal in most embodiments. More preferably however, the camshaft driving the cam that actuates the displacement of the housing furthermore drives a second cam that actuates the cutting means in these embodiments.

In a preferred embodiment, at least one of the holding means, preferably all of the holding means, comprises a suction means configured to exert a negative pressure from at least part of the holding means, to hold the wrapped strip. This can be embodied by the holding means having openings along its mantle and whereby a negative pressure is achieved in the holding means to hold the wrapped strip through the openings.

In a preferred embodiment, the strip is provided in a first position before the embracing means act upon the strip, whereby the strip in said first position lies in a generally parallel plane with respect to the conveyance direction of the conveying means.

In a third aspect, the invention provides a tampon packaging manufacturing apparatus comprising one or more holding means, an apparatus for wrapping a strip of film around a holding means as described in this document, and one or more sealing means for sealing a strip of film wrapped around the holding means to manufacture an elongate, preferably essentially cylindrical, tampon packaging.

The invention is further described by the following non-limiting examples which further illustrate the invention, and are not intended to, nor should they be interpreted to, limit the scope of the invention.

The present invention will be now described in more details, referring to examples that are not limitative.

### Examples

### Example 1: Tampon packaging manufacturing apparatus

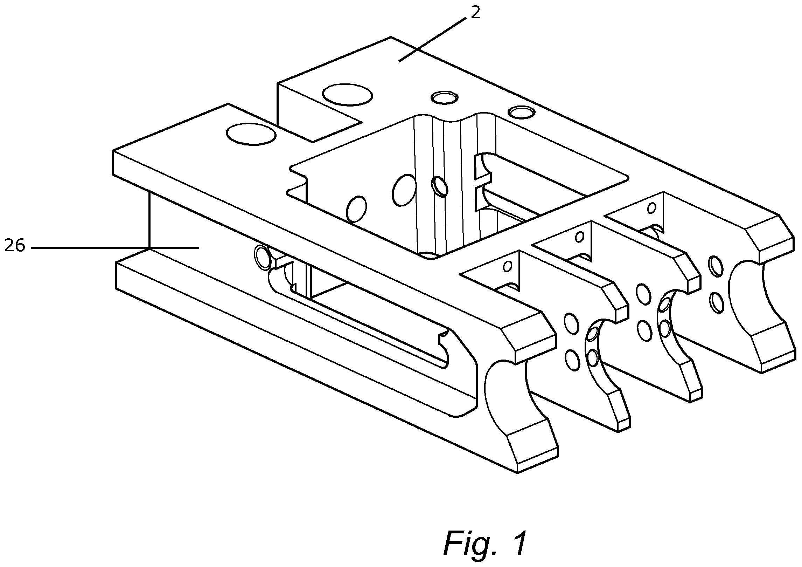

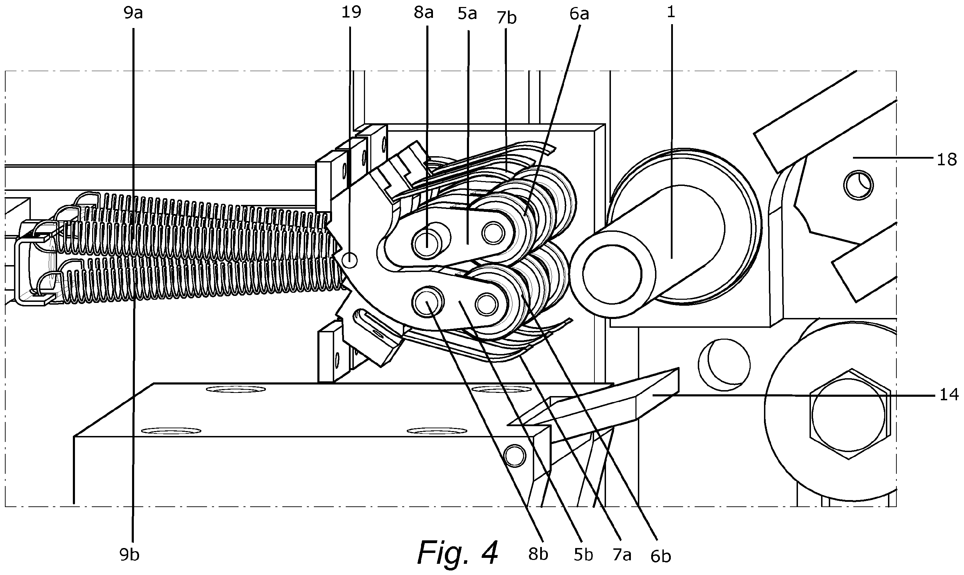

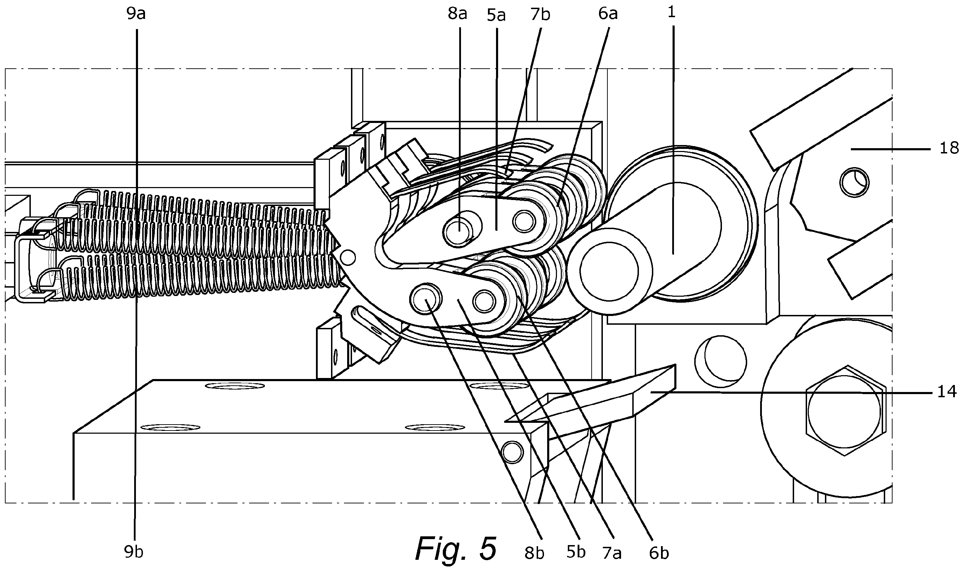

In FIG. 1 and 3-6, part of a tampon packaging apparatus is shown, more specifically the components where the film to form the packaging is essentially cut, applied and heat-sealed into a tubular packaging. FIG. 4 shows an internal view of the sets of embracing means (4a, 4b) with a holding means (1), whereby the sets of embracing means (4a, 4b) are in its first position, in so-called standby mode. The apparatus comprises a housing (2) mounted on a support (3), and two sets of embracing means (4a, 4b), wherein the sets of embracing means are an upper set of embracing means (4a) and a lower set of embracing means (4b). The sets (4a, 4b) each comprise one or more rotatable embracing means (5a, 5a', 5a", ... and 5b, 5b', 5b", ...) (in this case generally shaped as an "L" or "C", though this in no way limits the invention and other designs are very well conceivable), preferably 3 although a different number of embracing means would be possible such as 1, 2, 4, 5, 6, 7, 8, 10, 15, 20 or number therebetween, and perhaps even more suitable for certain applications (more elongate objects could require more embracing means (5a, 5a', 5a", ... and 5b, 5b', 5b", ...) per set, differently shaped holding means (1) could require more or less embracing means and even a precise placement of some of the embracing means). The embracing means (5a, 5a', 5a", ... and 5b, 5b', 5b", ...) of each (4a, 4b) set are mounted to rotate or pivot around a set shaft (8a, 8b), namely an upper set shaft (8a) and a lower set shaft (8b), but are preferably configured so that the embracing means (5a, 5a', 5a", ... and 5b, 5b', 5b", ...) can rotate independently from each other. The set shafts (8a, 8b) both are generally parallel to a presented holding means (1) (and to each other). Each of the embracing means (5a, 5a', 5a", ... and 5b, 5b', 5b", ...) preferably is connected to the housing (2) by a lever spring (9a, 9b) (optionally several lever springs (9a, 9b) for one embracing means), preferably whereby the lever spring (9a, 9b) is attached to the embracing means via an embracing means shaft (19) of said embracing means. The lever spring (9a, 9b) exerts a torque on the embracing means when it is not in its original position, rotating it back into the original position as depicted in FIG. 4-6. The term "original position" refers to a mode wherein the embracing means are at rest and are not experiencing torque from the lever springs (9a, 9b). The longitudinal axis of the lever springs (9a, 9b) of one set of embracing means (4a, 4b) is slightly tilted to the other set as can be seen in FIG. 4-5, which allows for a more convenient design where the lever springs (9a, 9b) will not interfere with each other and can be attached to the housing (2) with more ease as there is more space available for attachment.

At a first extremal end of the embracing means (5a, 5a', 5a", ... and 5b, 5b', 5b", ...), the embracing means comprise one or more pressing means (6), preferably smooth at least on the side that is directed towards the holding means (1). In this example, the pressing means (6) comprises one or more rollers (6) (preferably ball bearings), which are mounted at the first extremal end of the embracing means (5a, 5a', 5a", ... and 5b, 5b', 5b", ...) with a rotational axis that is generally parallel to the set shafts (8a, 8b). Although a smooth surface would also produce the desired effect of pressing a strip against the holding means (1), the use of a roller (6), and thereby a rotating (or at least rotatable) pressing means (6), is preferred as this will cause less friction which could damage the film. As noted in previous paragraph, the lever springs (9a, 9b) cause the embracing means (5a, 5a', 5a", ... and 5b, 5b', 5b", ...) to attempt to return to their original position. When the rollers (6) move over the holding means (1), they are rotated from their original position in order to follow the mantle of the holding means (1). This causes the lever springs (9a, 9b) to exert torque on the embracing means (5a, 5a', 5a", ... and 5b, 5b', 5b", ...), thus pressing the rollers (6) against the mantle of the holding means (1) and exerting a force on the holding means (1) that is directed inwardly, and in this case radially inwardly (however, this would depend on the shape of the holding means (1), generally the force would be directed inwardly along the normal on the mantle of the holding means (1) where the roller (6) touches the holding means (1)). This ensures that the strips would be wrapped firmly and tightly around the holding means (1). Also, the number of rollers (6) is not limited to that of the example and one can easily imagine variations with more or less rollers (6), for instance 1, 2, 4, 5, 6, 7, 8, 9, 10, 12, 15, 20, 25 or even more. Furthermore, it is not necessary that the number of rollers matches the number of embracing means. However, this is preferably so.

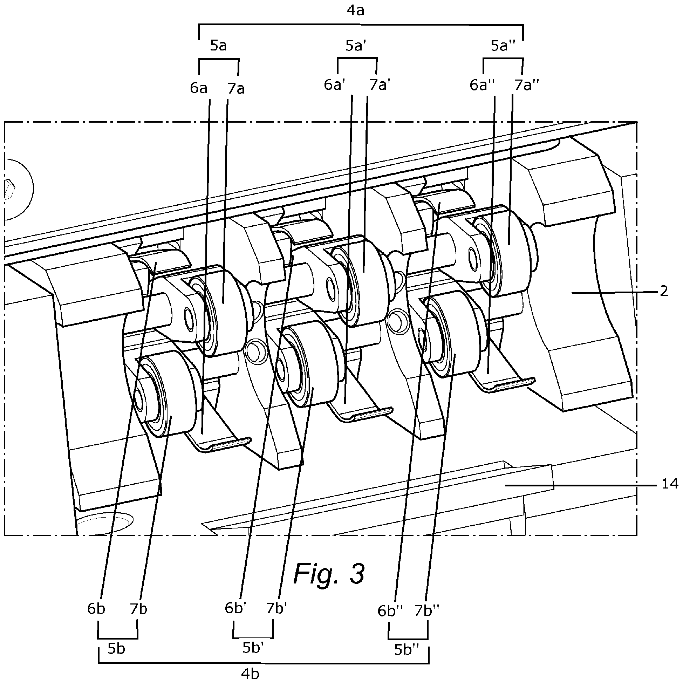

At the second extremal end (opposite to the first extremal end) of the embracing means (5a, 5a', 5a", ... and 5b, 5b', 5b", ...), the embracing means comprise one or more wrapping means (7), in this case finger-like extensions, preferably 1 per embracing means and preferably (slightly) flexible. The extensions (7) of each set preferably extend beyond the rollers (6) of the other set of embracing means. The fingers of the upper set of embracing means are located below the lower set of embracing means (4b), the fingers of the lower set of embracing means are located above the upper set of embracing means (4a). The extensions (7) are preferably bent at the distal end of the extension (7) and bend towards the extensions of the other set of embracing means, as can be seen in FIG. 3-5, in order to better be able to fold a strip of film around the holding means (1) and by the springs. It is clear in FIG. 4-5 that, when the housing (2), and therefore the embracing means (5a, 5a', 5a", ... and 5b, 5b', 5b", ...), is displaced towards the holding means (1) so that the rollers (6) move over the surface of the holding means (1), thereby causing the embracing means (5a, 5a', 5a", ... and 5b, 5b', 5b", ...) to rotate around the upper set shaft (8a), that the rollers (6) of the upper set (4a) will be pushed slightly backward and upward with respect to the housing (2). This movement can continue until the rollers (6) reach the highest point of the holding means (1) (to say, of the circular cross-section of the holding means (1)). Due to this rotation, the extensions (7) of the upper set (4a) (said extensions are, as said, at the bottom in FIG. 4-5) will be pushed forward (to the right) and more importantly, reach around the holding means (1) to fold an interposed strip around the holding means (1). The same principle applies to the extensions (7) of the lower set (4b) moving by the interaction of the rollers (6) of the lower set (4b) with the holding means (1). Again, the number of extensions (7) is not limited to that of the example and one can easily imagine variations with more or less extensions (7), for instance 1, 2, 4, 5, 6, 7, 8, 9, 10, 12, 15, 20, 25 or even more. Furthermore, it is not necessary that the number of extensions matches the number of rollers and/or of embracing means. However, this is preferably so.

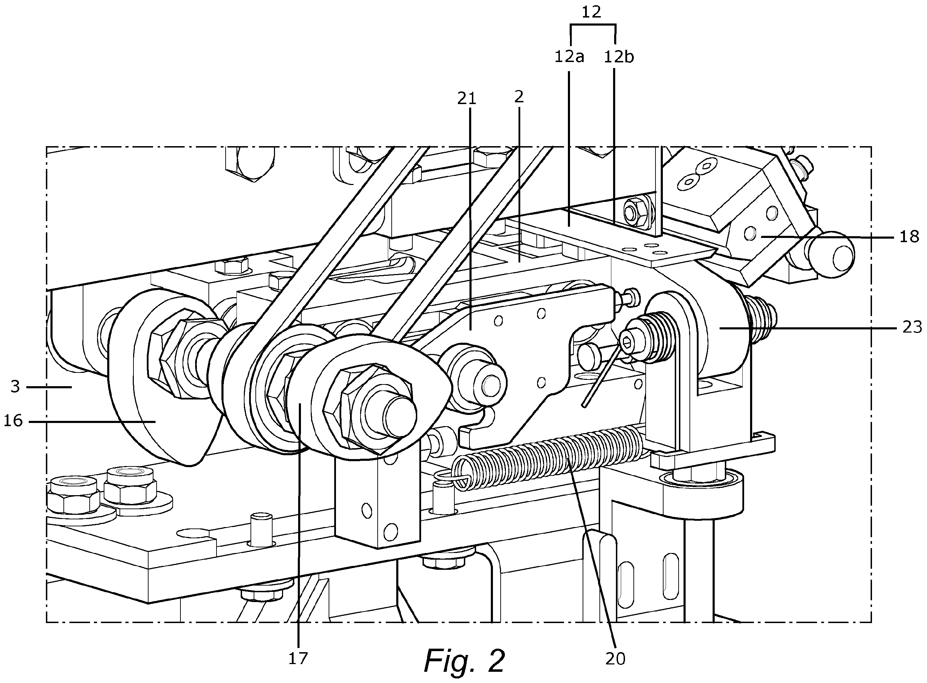

A possible embodiment of a housing (2) can be seen in FIG. 1. The embracing means (5a, 5a', 5a", ... and 5b, 5b', 5b", ...), and thereby the wrapping and pressing means (6) are rotatably attached to the housing (2) by the set shafts (8a, 8b). The embracing means are furthermore attached to the housing (2) by the lever springs (9a, 9b) which results in an original position of the embracing means at rest as in FIG. 4-5. When the housing (2) is displaced forward, forward being toward the holding means (1), so do the sets of embracing means (4a, 4b) which are attached to the housing (2) by the set shafts (8a, 8b) and so do the rollers (6) and the extensions (7) which are attached to the embracing means . When the rollers (6) of the embracing means make contact with the holding means (1), they are initially pushed outwards to follow the shape of the holding means (1), thereby rotating the sets of embracing means (4a, 4b) and the embracing means (5a, 5a', 5a", ... and 5b, 5b', 5b", ...) thereof, while still moving further. The housing (2) is adapted to that it can be moved forward (toward the conveying means (11)/holding means (1)) from its first position and backward (back toward the first position). The forward movement can for instance be induced by a rotatable cam (16) of which the rotation speed can be adjusted. The cam (16) is uneven (in this case 1 eccentricity, but could however also have several eccentricities), as can be seen in FIG. 2, so that by rotating, it induces the movement of the housing (2), and thus the wrapping means (7). The backward movement of the housing (2) can be induced by a number of returning means (10), preferably whereby the returning means (10) are spring elements (10). The number of spring elements (10) can for instance be 1, 2, 3, 4, 6, 8, 10, 15, 20 or other, and said spring elements (10) pull the housing (2) back toward the first position once the cam (16) no longer pushes the housing (2) forward. The rotation speed of the cam (16) can be adjusted so that the housing (2) (and wrapping means) is displaced forward towards the conveying means (11) when a holding means (1) arrives in front of the embracing means.

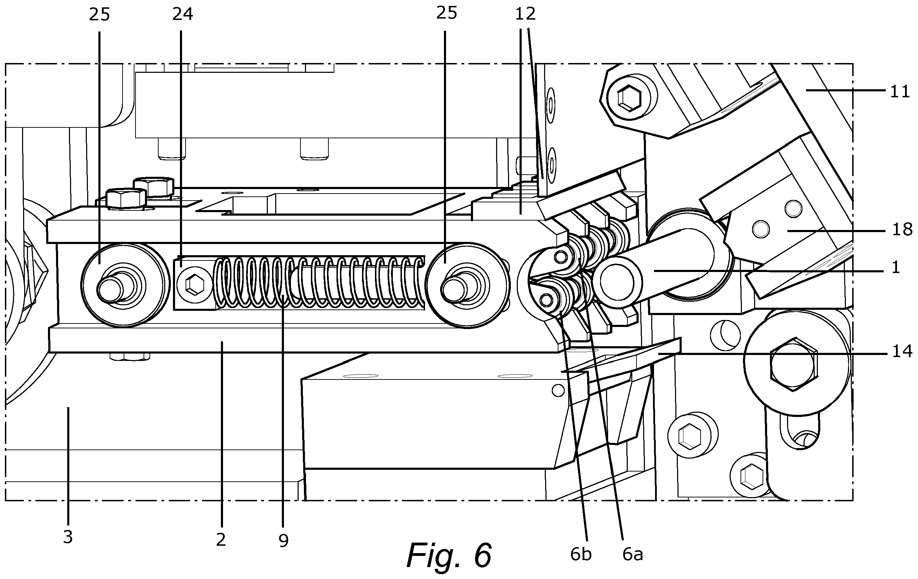

In a more detailed description of a possible returning mechanism for the housing, the housing (2) moves over a transport means, for instance ball bearings (25) provided on either side of the housing (2) in order to ensure a consistent path whereby the ball bearings (25) are attached to the support (3). The housing (2) has a slit (16) along the sides (lengthwise) on both sides, as can be seen on one side in FIG. 1 and FIG. 6, in which the ball bearings (25) fit, thereby supporting the housing (2) while allowing displacement of the housing (2) along a displacement axis. The spring attachment elements (24), for attaching the spring elements (10) to the housing (2), move in concert with the housing (2). As can be seen in FIG. 6, where the housing (2) has not been displaced towards the holding means (1), the spring elements (10) are not compressed. However, as the housing (2) would be pushed towards the holding means (1) by the cam (16), the spring attachment elements (24) would move along with the housing (2), thereby compressing the spring elements (10). Once the cam (16) no longer causes the displacement of the housing (2), the compressed spring elements (10) will push the spring attachment elements (24), and thereby the housing (2) back to its original position. This is however just one of the possible embodiments and in no way to be considered as a restriction.

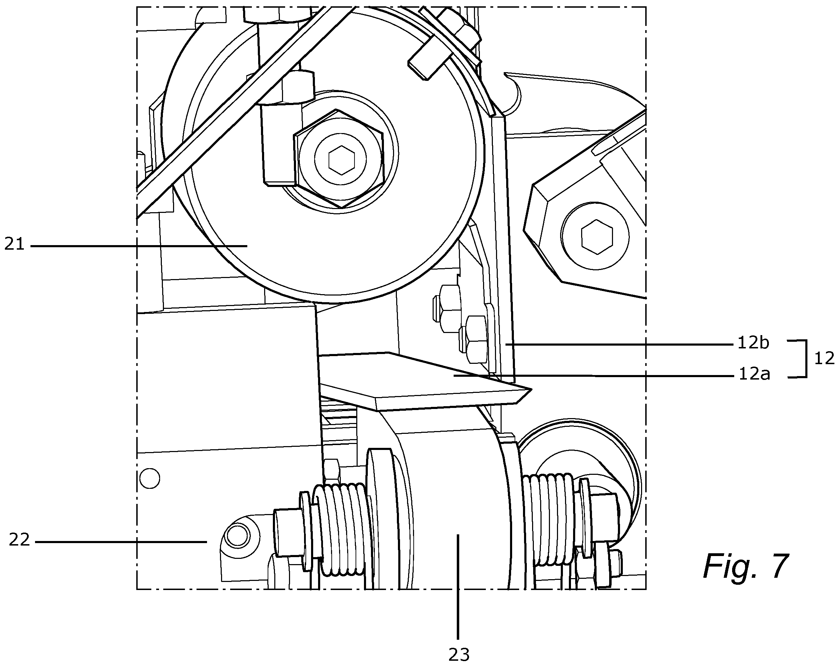

The tampon packaging manufacturing apparatus can furthermore comprise a film providing means (22) for periodically providing a length of film in a stop-go process, between the wrapping means (7) and the holding means (1) that is present in front of the wrapping means (7). Preferably this process is executed abruptly so as not to slow down the process, or more precisely, faster than a next holding means (1) is provided to the wrapping means (7). Again this speed can be adjusted to match the periodicity of the holding means (1) and the displacements of the wrapping means (7) so that a new sheet is provided with the length of a strip (slightly more than the circumference of a holding means (1)). The film providing means (22) can at least partly be seen in FIG. 7.

The tampon packaging manufacturing apparatus can furthermore comprise a cutting (12) means, as shown in FIG. 2 and 7, which works in concert with the film providing means (22) in that when the film providing means (22) has provided a sheet of film with the length of a strip, the cutting means (12) cuts the provided sheet, thereby creating a separated strip of film which is held between the wrapping means (7) and the presented holding means (1). The cutting means (12) can be actuated by a second uneven cam (17), similar to the cam (16) used to displace the housing (2) and the sets of embracing means (4a, 4b). The cam (17) displaces a first intermediary element (21) which in turn displaces the arm (23) to which the blade (12a) of the cutting means (12) is attached, as can be seen in FIG. 2 and in more detail in FIG. 7. The blade (12a) a length of film against a second part of the cutting means (12b). The cutting means (12) preferably operate similar to a pair of scissors, wherein the blade (12a) visible in FIG. 7, cuts through the film at one point which point moves along the cutting line as the cut is made. More precisely, in FIG. 2, the second cam (17) has displaced the intermediary element (21) and therefore the arm (23) of the cutting means (12) is in a position in which the cutting of the strip has already been executed (if film were provided). The cutting means (12) can then be returned to its original position by a blade spring (20) which retracts (indirectly through the support of the arm (23)) the blade (12a) of the cutting means (12) when the uneven cam no longer pushes the blade (12a). As the embracing means and the cutting means (12) would ideally operate at the same frequency, the cam (16) of the housing (2) and thus of the embracing means (4a, 4b) and the cam (17) of the cutting means (12) could be driven by a single actuator to ensure a perfectly equal period. The apparatus furthermore may comprise a supporting means (14) for supporting said separated strip. This can be a small horizontally placed flap on which the strip can rest. As the time between the cutting of the strip and the wrapping of the strip is very short, the supporting means (14) needs to be only basic and is designed to temporarily prevent the strip from falling or slipping away.

The tampon packaging manufacturing apparatus further comprises a conveying means (11) with one or more holding means (1). The number of holding means (1) can vary, for instance 1, 2, 3, 4, 5, 6, 7, 8, 9, 10, 12, 15, 20, 24, 30, 40, 50 or more and number therein between. Preferably the holding means (1) are disposed intermittently over the conveying means (11) over equal distances. In this case, the conveying means (11) is a wheel rotatable around a central axis, with a number of holding means (1), in this example elongate bars with a slightly larger radius and length than the tampons. The separate holding means (1) are mounted at the edge of the wheel at one side of the wheel, and with the longitudinal axis of the holding means (1) substantially perpendicular to the plane of the wheel.

In FIG. 6, a fixating means (18) is visible that is adapted to clamp down on the holding means (1) when the strip of film is wrapped around the holding means (1). Optionally, there is only of said fixating means (18) which remains stationary and is adapted to fixate a strip of film to whichever holding means (1) is presented to the sets of embracing means (4a, 4b). Alternatively, the conveying means (11) comprises a plurality of said fixating means (18), preferably one for each holding means (1), whereby each fixating means (18) is adapted to clamp down a strip of film on the related holding means (1). More preferably, the fixating means (18) comprises a sealing element which can be adapted so it can both clamp down a wrapped strip of film to the holding means (1) and seal the strip of film, for instance via heat-sealing.

An example for the apparatus wherein the holding means are moved towards the embracing means (instead of the other way around as in the above example) is not provided, however, by obvious adaptations of the above example, the average skilled person in the art could easily provide such an apparatus. For instance, the housing would be part of the support, while the configuration of the embracing means (and sets) could remain quite similar, as it is the displacement at the hands of the holding means that actuates the wrapping movement, not the displacement of the housing per se. Therefore, only a holding means that is movable towards the embracing means would need to be provided, which should easily be accomplished by the average skilled person.

It is supposed that the present invention is not restricted to any form of realization described previously and that some modifications can be added to the presented example of fabrication without reappraisal of the appended claims. For example, the amount of certain elements or features is not restricted to the number indicated in this text, but to all logical variations which would cause the desired effect the invention intends. Furthermore, the present invention has been described referring to the manufacturing of packagings for tampons, but it is clear that the invention can be applied to packagings for cigarettes for instance or for lipstick, lip balm, and generally other tube-shaped products. However, it is to be noted that the invention can be expanded further as the shape of the packaging can easily be altered to different shapes, for instance coni, pyramids, cuboids or combinations of several different elements.

## Claims

1. Method for wrapping a strip of film around a holding means, comprising the steps of:
a. providing a strip, preferably a quadrilateral strip and more preferably a rectangular strip, preferably of heat-sealable film;
b. providing an elongate holding means having a longitudinal axis parallel to the strip; **characterised by**
c. displacing the strip along a displacement axis essentially perpendicular to said longitudinal axis towards the holding means and/or displacing the holding means along a displacement axis essentially perpendicular to said longitudinal axis towards the strip;
d. pressing the strip against the holding means between a first end of the strip and a second end of the strip;
e. while the strip is pressed against the holding means, wrapping the first end of the strip around the holding means in a first rotational direction and wrapping the second end of the strip around the holding means in a second rotational direction opposite to the first rotational direction;
f. optionally immobilizing the wrapped strip of film with respect to the holding means; and
g. optionally sealing, preferably heat-sealing, an overlap of the first and the second end of the strip.

2. Method for wrapping a strip of film around a holding means, according to the preceding claim 1, whereby wrapping of the first end of the strip around the holding means and wrapping of the second end of the strip around the holding means is executed so that at least part of the first end overlaps with at least part of the second end of the strip, **characterized in that** the at least part of the second end of the strip is wrapped between the holding means and the at least part of the first end of the strip.

3. Method for wrapping a strip of film around a holding means, according to any one of the preceding claims 1 or 2, whereby the strip is pressed against the holding means between a first end of the strip and a second end of the strip in a first pressing area and a second pressing area, **characterized in that** the first pressing area continuously moves over the holding means during the wrapping of the first end of the strip and the second pressing area continuously moves over the holding means during the wrapping of the second end of the strip.

4. Method for wrapping a strip of film around a holding means, according to any one of the preceding claims 1 to 3, **characterized in that** in step c the strip is displaced along the displacement axis essentially perpendicular to the longitudinal axis towards the holding means, preferably wherein the holding means remains stationary, during the step of displacing the strip, pressing the strip and wrapping the strip, and preferably during the step of sealing the strip and/or wherein in step c the holding means are displaced along a displacement axis essentially perpendicular to said longitudinal axis towards the strip, preferably wherein the housing remains stationary, during the step of displacing the strip, pressing the strip and wrapping the strip, and preferably during the step of sealing the strip.

5. Apparatus for wrapping a strip of film around an elongate holding means (1) to manufacture an elongate, preferably essentially cylindrical, tampon packaging, comprising a support (3) and a housing (2), the apparatus further comprising a first set of embracing means (4a) and a second set of embracing means (4b), the first set of embracing means being mounted rotatably onto the housing (2) around a first pivot axis (8a) and the second set of embracing means being mounted rotatably onto the housing around a second pivot axis (8b) which is essentially parallel to the first pivot axis;
wherein the housing (2) is displaceably mounted on the support (3) and is reversibly movable with respect to said support along a displacement axis essentially towards the holding means (1) for pushing the strip of film towards the holding means by at least one of said embracing means (4a, 4b), said displacement axis essentially perpendicular to said first and second pivot axes (8a, 8b), thereby defining a pinching direction essentially perpendicular to said displacement axis and to said first and second pivot axes; and/or wherein the holding means (1) is reversibly movable along a displacement axis essentially towards the housing (2) for pushing the strip of film towards the embracing means (4a, 4b), said displacement axis essentially perpendicular to said first and second pivot axes (8a, 8b), thereby defining a pinching direction essentially perpendicular to said displacement axis and to said first and second pivot axes;
wherein each set of embracing means (4a, 4b) comprises at least one embracing means (5a, 5a', 5a", 5b, 5b', 5b") comprising:
- a pressing means (6) for pressing the strip of film between the embracing means and the holding means; and
- a wrapping means (6, 7) for wrapping the strip of film around the holding means,
wherein the wrapping means (6a, 6a', 6a", 7a, 7a', 7a") of the embracing means of the first set of embracing means is configured to execute a wrapping movement for wrapping the strip of film around the holding means (1) in a first rotational direction while the embracing means are being displaced and the pressing means are pressed against the holding means, and
wherein the wrapping means (6b, 6b', 6b", 7b, 7b', 7b") of the embracing means of the second set of embracing means is configured to execute a wrapping movement for wrapping the strip of film around the holding means in a second rotational direction opposite to the first rotational direction, while the embracing means are being displaced and the pressing means are pressed against the holding means.

6. Apparatus according to the preceding claim 5, wherein the pressing means of at least one embracing means of the first set of embracing means is displaceable along at least the pinching direction with respect to the housing and whereby the embracing means is configured so that a displacement with respect to the housing of said pressing means of the at least one embracing means of the first set of embracing means along at least the pinching direction, actuates the wrapping movement of the wrapping means of the at least one embracing means of the first set of embracing means, preferably wherein the pressing means of all the embracing means of the first set of embracing means are displaceable along at least the pinching direction with respect to the housing and whereby all the embracing means are configured so that a displacement with respect to the housing of said pressing means of one or more of the embracing means of the first set of embracing means along at least the pinching direction, actuates the wrapping movement of the wrapping means of said one or more of the embracing means of the first set of embracing means;
and/or wherein the pressing means of at least one embracing means of the second set of embracing means is displaceable along at least the pinching direction with respect to the housing and whereby the embracing means is configured so that a displacement with respect to the housing of said pressing means of the at least one embracing means of the second set of embracing means along at least the pinching direction, actuates the wrapping movement of the wrapping means of the at least one embracing means of the second set of embracing means, preferably wherein the pressing means of all the embracing means of the second set of embracing means are displaceable along at least the pinching direction with respect to the housing and whereby all the embracing means are configured so that a displacement with respect to the housing of said pressing means of one or more of the embracing means of the second set of embracing means along at least the pinching direction, actuates the wrapping movement of the wrapping means of said one or more of the embracing means of the second set of embracing means.

7. Apparatus according to any one of the preceding claims 5 or 6, wherein the pressing means of at least one embracing means of the first set of embracing means is adapted to continuously move over the holding means in the second rotational direction while the at least one embracing means of the first set of embracing means is being displaced, preferably wherein the pressing means of all the embracing means of the first set of embracing means are adapted to continuously move over the holding means in the second rotational direction while the embracing means of the first set of embracing means are being displaced;
and/or wherein the pressing means of at least one embracing means of the second set of embracing means is adapted to continuously move over the holding means in the first rotational direction while the at least one embracing means of the second set of embracing means is being displaced, preferably wherein the pressing means of all the embracing means of the second set of embracing means are adapted to continuously move over the holding means in the first rotational direction while the embracing means of the second set of embracing means are being displaced.

8. Apparatus according to any one of the preceding claims 5 to 7, whereby the first set of embracing means is capable of wrapping a first end of the strip around the holding means in the first rotational direction and whereby the second set of embracing means is capable of wrapping a second end of the strip opposite to the first end of the strip, around the holding means in the second rotational direction, whereby at least part of the wrapped first end of the strip and at least part of the wrapped second end of the strip are superimposed, and is **characterized in that** the first set of embracing means and the second set of embracing means are configured with respect to each other so that the superimposed part of the first end of the strip is positioned between the holding means and the superimposed part of the second strip.

9. Apparatus according to any one of the preceding claims 5 to 8, comprising:
a. a film providing means for providing the film;
b. a cutting means for cutting the film, thereby forming the strip of film and providing said strip of film between the holding means and the first and second set of embracing means; and
c. optionally a supporting means for supporting the strip of film between the holding means and the first and second set of embracing means.

10. Apparatus according to any one of the preceding claims 5 to 9, comprising a conveying means which comprises one or more of said holding means and is configured to provide the holding means in a stop-go process to a stationary position which is essentially distanced from the first and the second set of embracing means along the displacement axis, preferably whereby the conveying means is a conveying wheel capable of rotating with a multitude of said holding means equidistantially disposed along the edge of said conveying wheel.

11. Apparatus according to any one of the preceding claims 5 to 10, whereby the pressing means of at least one embracing means of the first and/or the second set of embracing means comprises one or more rollers, preferably whereby the pressing means of all the embracing means of the first and the second set of embracing means comprise one or more rollers, whereby the one or more rollers are adapted to continuously move over the holding means while the housing is being displaced along the displacement axis and while the one or more rollers press against the holding means.

12. Apparatus according to any one of the preceding claims 5 to 11, whereby the wrapping means of at least one embracing means of the first and/or the second set of embracing means comprises one or more extensions, preferably whereby the wrapping means of all of the embracing means of the first and the second set of embracing means comprises one or more extensions, whereby the one or more extensions are adapted to execute the wrapping movement for wrapping the strip of film around the holding means while the housing is being displaced along the displacement axis and while the one or more pressing means press against the holding means.

13. Apparatus according to any one of the preceding claims 5 to 12, whereby at least one, preferably all, of the embracing means of the first and/or of the second set are connected to the housing by one or more springs and whereby the springs are configured to actuate the pressing of the pressing means against the holding means.

14. Apparatus according to any one of the preceding claims 5 to 13, the wrapping means are adapted to wrap the strip of film around the holding means so that a first end of the strip and an opposite, second end of the strip of film are superimposed, and whereby the apparatus comprises one or more fixating means adapted to clamp the superimposed ends of the wrapped strip of film and/or seal, preferably heat-seal, said superimposed ends of the wrapped strip of film, preferably whereby the apparatus comprises one of said fixating means for each of the holding means.

15. Tampon packaging manufacturing apparatus comprising one or more holding means, an apparatus for wrapping a strip of film around an elongate holding means according to any one of the claims 5 to 14, and a sealing means for sealing a strip of film wrapped around the holding means to manufacture an elongate, preferably essentially cylindrical, tampon packaging.

## Patentansprüche

1. Verfahren zum Wickeln eines Folienstreifens um ein Haltemittel, umfassend die Schritte vom:
a. Bereitstellen eines Streifens, vorzugsweise eines viereckigen Streifens, und mehr bevorzugt ein rechteckiger Streifen, vorzugsweise einer heißsiegelbaren Folie;
b. Bereitstellen eines länglichen Haltemittels mit einer Längsachse parallel zu dem Streifen;
**gekennzeichnet durch**
c. Verschieben des Streifens entlang einer Verschiebungsachse, die im Wesentlichen senkrecht zu der Längsachse in Richtung des Haltemittels verläuft, und/oder Verschieben des Haltemittels entlang einer Verschiebungsachse, die im Wesentlichen senkrecht zu der Längsachse in Richtung des Streifens verläuft;
d. Drücken des Streifens gegen das Haltemittel zwischen einem ersten Ende des Streifens und einem zweiten Ende des Streifens;
e. während der Streifen gegen das Haltemittel gedrückt wird, das Umwickeln des ersten Endes des Streifens um das Haltemittel in eine erste Drehrichtung und Umwickeln des zweiten Endes des Streifens um das Haltemittel in eine zweite Drehrichtung entgegengesetzt zu der ersten Drehrichtung;
f. optionales Immobilisieren des umwickelten Folienstreifens in Bezug auf das Haltemittel; und
g. optionales Versiegeln, vorzugsweise Heißsiegeln, der Überlappung des ersten und des zweiten Endes des Streifens.

2. Verfahren zum Wickeln eines Folienstreifens um ein Haltemittel, nach dem vorhergehenden Anspruch 1, wobei das Wickeln des ersten Endes des Streifens um das Haltemittel und das Wickeln des zweiten Endes des Streifens um das Haltemittel so ausgeführt wird, dass sich mindestens ein Teil des ersten Endes mit mindestens einem Teil des zweiten Endes des Streifens überlagert, **dadurch gekennzeichnet, dass** mindestens der Teil des zweiten Endes des Streifens zwischen dem Haltemittel und mindestens dem Teil des ersten Endes des Streifens gewickelt ist.

3. Verfahren zum Wickeln eines Folienstreifens um ein Haltemittel, nach einem der vorhergehenden Ansprüche 1 oder 2, wobei der Streifen zwischen einem ersten Ende des Streifens und einem zweiten Ende des Streifens in einem ersten Andrückbereich und einem zweiten Andrückbereich gegen das Haltemittel gedrückt wird, **dadurch gekennzeichnet, dass** sich der erste Andrückbereich während des Umwickelns des ersten Endes des Streifens kontinuierlich über das Haltemittel hinweg bewegt und sich der zweite Andrückbereich während des Umwickelns des zweiten Endes des Streifens kontinuierlich über das Haltemittel hinweg bewegt.

4. Verfahren zum Wickeln eines Folienstreifens um ein Haltemittel, nach einem der vorhergehenden Ansprüche 1 bis 3, **dadurch gekennzeichnet, dass** in Schritt c der Streifen entlang der Verschiebungsachse im Wesentlichen senkrecht zur Längsachse in Richtung des Haltemittels verschoben wird, vorzugsweise wobei sich das Haltemittel während des Schritts vom Verschieben des Streifens, Andrücken des Streifens und Umwickeln den Streifens, und vorzugsweise während des Schritts vom Versiegeln des Streifens stationär bleibt und/oder wobei in Schritt c das Haltemittel entlang einer im Wesentlichen senkrechten Verschiebungsachse zu der Längsachse in Richtung des Streifens verschoben wird, vorzugsweise wobei sich das Gehäuse während des Schritts vom Verschieben des Streifens, Andrücken des Streifens und Umwickeln des Streifens, und vorzugsweise während des Schritts vom Versiegeln des Streifens stationär bleibt.

5. Vorrichtung zum Wickeln eines Folienstreifen um ein längliches Haltemittel (1) zur Herstellung einer länglichen, vorzugsweise im Wesentlichen zylindrischen, Tamponverpackung, umfassend einen Träger (3) und ein Gehäuse (2), die Vorrichtung ferner umfassend einen ersten Satz Umfassungsmittel (4a) und einen zweiten Satz Umfassungsmittel (4b), wobei der erste Satz Umfassungsmittel um eine erste Schwenkachse (8a) drehbar auf dem Gehäuse (2) montiert ist und der zweite Satz Umfassungsmittel um eine zweite Schwenkachse(8b), die im Wesentlichen parallel zur ersten Schwenkachse ist , drehbar auf dem Gehäuse montiert ist;
wobei das Gehäuse (2) auf dem Träger (3) verschiebbar montiert ist und in Bezug auf den Träger entlang einer Verschiebungsachse im Wesentlichen in Richtung des Haltemittels (1) umkehrbar beweglich ist, um den Folienstreifen gegen das Haltemittel zu drücken durch mindestens eines der Umfassungsmittel (4a, 4b), wobei die Verschiebungsachse im Wesentlichen senkrecht zu den ersten und zweiten Schwenkachsen (8a, 8b) ist, wodurch eine Einklemmrichtung bestimmt wird, die im Wesentlichen senkrecht zur Verschiebungsachse und zu den ersten und zweiten Schwenkachsen ist; und/oder wobei das Haltemittel (1) entlang einer Verschiebungsachse im Wesentlichen in der Richtung des Gehäuses (2) umkehrbar beweglich ist, um den Folienstreifen in der Richtung der Umfassungsmittel (4a, 4b) zu drücken, wobei die Verschiebungsachse im Wesentlichen senkrecht zu den ersten und zweiten Schwenkachsen (8a, 8b) ist, wodurch eine Einklemmrichtung bestimmt wird, die im Wesentlichen senkrecht zu der Verschiebungsachse und zu den ersten und zweiten Schwenkachsen ist; wobei jeder Satz Umfassungsmittel (4a, 4b) mindestens ein Umfassungsmittel (5a, 5a', 5a", 5b, 5b', 5b") umfasst, umfassend:
- ein Andrückmittel (6) zum Andrücken des Folienstreifens zwischen das Umfassungsmittel und das Haltemittel; und
- ein Umwickelmittel (6, 7) zum Umwickeln des Folienstreifen um das Haltemittels,
wobei das Umwickelmittel (6a, 6a', 6a", 7a, 7a', 7a") des Umfassungsmittels des ersten Satzes Umfassungsmittel so konfiguriert ist, dass es eine Umwickelbewegung ausführt, um den Folienstreifen um das Haltemittel (1) in einer ersten Drehrichtung zu wickeln, während die Umfassungsmittel verschoben werden und die Andrückmittel gegen das Haltemittel gedrückt werden, und wobei das Umwickelmittel (6b, 6b', 6b", 7b, 7b', 7b") des Umfassungsmittels des zweiten Satzes Umfassungsmittel so konfiguriert ist, dass es eine Umwickelbewegung zum Wickeln des Folienstreifens um das Haltemittel in einer zweiten Drehrichtung entgegen der ersten Drehrichtung ausführt, während die Umfassungsmittel verschoben werden und die Andrückmittel gegen das Haltemittel gedrückt werden.

6. Vorrichtung nach dem vorhergehenden Anspruch 5, wobei das Andrückmittel von mindestens einem Umfassungsmittel des ersten Satzes Umfassungsmittel entlang mindestens der Einklemmrichtung in Bezug auf das Gehäuse verschiebbar ist und wobei das Umfassungsmittel so konfiguriert ist, dass eine Verschiebung in Bezug auf das Gehäuse des Andrückmittels von mindestens einem Umfassungsmittel des ersten Satzes Umfassungsmittel entlang mindestens der Einklemmrichtung die Umwickelbewegung des Umwickelmittels von mindestens einem Umfassungsmittel des ersten Satzes Umfassungsmittel auslöst, wobei vorzugsweise die Andrückmittel aller Umfassungsmittel des ersten Satzes Umfassungsmittel entlang mindestens der Einklemmrichtung in Bezug auf das Gehäuse verschiebbar sind und wobei alle Umfassungsmittel so konfiguriert sind, dass eine Verschiebung in Bezug auf das Gehäuse des Andrückmittels von einem oder mehreren der Umfassungsmittel des ersten Satzes Umfassungsmittel entlang mindestens der Einklemmrichtung die Umwickelbewegung des Umwickelmittels des einen oder mehreren Umfassungsmittel des ersten Satzes Umfassungsmittel auslöst;
und/oder wobei das Andrückmittel von mindestens einem Umfassungsmittel des zweiten Satzes Umfassungsmittel entlang zumindest der Einklemmrichtung in Bezug auf das Gehäuse verschiebbar ist und wobei das Umfassungsmittel so konfiguriert ist, dass eine Verschiebung in Bezug auf das Gehäuse des Andrückmittels von mindestens einem Umfassungsmittel des zweiten Satzes Umfassungsmittel entlang zumindest der Einklemmrichtung die Umwickelbewegung des Umwickelmittels von mindestens einem Umfassungsmittel des zweiten Satzes Umfassungsmittel auslöst, wobei vorzugsweise die Andrückmittel aller Umfassungsmittel des zweiten Satzes Umfassungsmittel entlang mindestens der Einklemmrichtung in Bezug auf das Gehäuse verschiebbar sind und wobei alle Umfassungsmittel so konfiguriert sind, dass eine Verschiebung in Bezug auf das Gehäuse des Andrückmittels von einem oder mehreren Umfassungsmitteln des zweiten Satzes Umfassungsmittel entlang zumindest der Einklemmrichtung die Umwickelbewegung des Umwickelmittels von einem oder mehreren Umfassungsmitteln des zweiten Satzes Umfassungsmittel auslöst.

7. Vorrichtung nach einem der vorhergehenden Ansprüche 5 oder 6, wobei das Andrückmittel von mindestens einem Umfassungsm ittel des ersten Satzes Umfassungsmittel dazu eingerichtet ist, sich kontinuierlich während der Verschiebung von mindestens einem Umfassungsmittel des ersten Satzes Umfassungsmittel in der zweiten Drehrichtung über die Haltemittel zu bewegen, vorzugsweise wobei die Andrückmittel aller Umfassungsmittel des ersten Satzes Umfassungsmittel dazu eingerichtet sind, sich während der Verschiebung der Umfassungsmittel des ersten Satzes Umfassungsmittel kontinuierlich in der zweiten Drehrichtung über die Haltemittel zu bewegen;
und/oder wobei das Andrückmittel von mindestens einem Umfassungsmittel des zweiten Satzes Umfassungsmittel dazu eingerichtet ist, sich kontinuierlich während der Verschiebung von mindestens einem Umfassungsmittel des zweiten Satzes Umfassungsmittel in der ersten Drehrichtung über das Haltemittel zu bewegen, vorzugsweise wobei die Andrückmittel aller Umfassungsmittel des zweiten Satzes Umfassungsmittel dazu eingerichtet sind, sich während der Verschiebung der Umfassungsmittel des zweiten Satzes Umfassungsmittel kontinuierlich in der ersten Drehrichtung über die Haltemittel zu bewegen.

8. Vorrichtung nach einem der vorhergehenden Ansprüche 5 bis 7, wobei der erste Satz Umfassungsmittel in der Lage ist, ein erstes Ende des Streifens in der ersten Drehrichtung um das Haltemittel zu wickeln, und wobei der zweite Satz Umfassungsmittel in der Lage ist, ein zweites Ende des Streifens gegenüber des ersten Endes des Streifens um das Haltemittel in der zweiten Drehrichtung zu wickeln, wobei sich zumindest ein Teil des umwickelten ersten Endes des Streifens und zumindest ein Teil des umwickelten zweiten Endes des Streifens überlagern, und **dadurch gekennzeichnet ist, dass** der erste Satz Umfassungsmittel und der zweite Satz Umfassungsmittel so zueinander konfiguriert sind, dass der überlagerte Teil des ersten Endes des Streifens zwischen dem Haltemittel und dem überlagerten Teil des zweiten Streifens positioniert ist.

9. Vorrichtung nach einem der vorhergehenden Ansprüche 5 bis 8, umfassend:
a. Ein folienbildendes Mittel zur Bereitstellung der Folie;
b. ein Schneidmittel zum Schneiden der Folie, wodurch der Folienstreifen gebildet wird und der besagte Folienstreifen zwischen dem Haltemittel und dem ersten und zweiten Satz Umfassungsmittel bereitgestellt wird; und
c. optional eine Stützeinrichtung zum Abstützen des Folienstreifens zwischen dem Haltemittel und dem ersten und zweiten Satz Umfassungsmittel.

10. Vorrichtung nach einem der vorhergehenden Ansprüche 5 bis 9, umfassend eine Fördereinrichtung, die ein oder mehreren Haltemittel umfasst, und dafür ausgelegt ist, die Haltemittel in einem Stopp-Go-Vorgang in eine feste Position zu bringen, die im Wesentlichen von dem ersten und zweiten Satz Umfassungsmittel entlang der Verschiebungsachse entfernt ist, vorzugsweise wobei die Fördereinrichtung ein Förderrad ist, das sich mit einer Vielzahl von Haltemitteln drehen kann, die äquidistanziell entlang der Kante des Förderrads angeordnet sind.

11. Vorrichtung nach einem der vorhergehenden Ansprüche 5 bis 10, wobei das Andrückmittel von mindestens einem Umfassungsmittel des ersten und/oder des zweiten Satzes Umfassungsmittel eine oder mehrere Rollen umfasst, vorzugsweise wobei die Andrückmittel aller Umfassungsmittel des ersten und des zweiten Satzes Umfassungsmittel eine oder mehrere Rollen umfassen, wobei die eine oder mehreren Rollen dazu eingerichtet sind, sich kontinuierlich über die Haltemittel zu bewegen, während das Gehäuse entlang der Verschiebungsachse verschoben wird und während eine oder mehrere Rollen gegen das Haltemittel drücken.

12. Vorrichtung nach einem der vorhergehenden Ansprüche 5 bis 11, wobei das Umwickelmittel von mindestens einem Umfassungsmittel des ersten und/oder des zweiten Satzes Umfassungsmittel eine oder mehrere Verlängerungen umfasst, vorzugsweise wobei das Umwickelmittel von allen Umfassungsmitteln des ersten und des zweiten Satzes Umfassungsmittel eine oder mehrere Verlängerungen umfasst, wobei die eine oder mehreren Verlängerungen dazu eingerichtet sind, die Umwickelbewegung zum Wickeln des Folienstreifens um das Haltemittel auszuführen, während das Gehäuse entlang der Verschiebungsachse verschoben wird und während ein oder mehrere Andrückmittel gegen das Haltemittel drücken.

13. Vorrichtung nach einem der vorhergehenden Ansprüche 5 bis 12, wobei mindestens ein, vorzugsweise alle Umfassungsmittel des ersten und/oder des zweiten Satzes durch eine oder mehrere Federn mit dem Gehäuse verbunden sind und wobei die Federn so konfiguriert sind, dass sie das Drücken der Andrückmittel gegen das Haltemittel auslösen.

14. Vorrichtung nach einem der vorhergehenden Ansprüche 5 bis 13, wobei die Umwickelmittel dazu eingerichtet sind, dass sie den Folienstreifen um das Haltemittel wickeln, sodass sich ein erstes Ende des Streifens und ein gegenüberliegendes, zweites Ende des Folienstreifens überlagern, und wobei die Vorrichtung ein oder mehrere Fixiermittel umfasst, die dazu eingerichtet sind, die überlagerten Enden des umwickelten Folienstreifens festzuklemmen und/oder zu versiegeln, vorzugsweise die überlagerten Enden des umwickelten Folienstreifens heiß zu versiegeln, vorzugsweise wobei die Vorrichtung ein der genannten Fixiermittel für jedes einzelne Haltemittel umfasst.

15. Vorrichtung zur Herstellung von Tamponverpackungen umfassend ein oder mehrere Haltemittel, eine Vorrichtung zum Wickeln eines Folienstreifens um ein längliches Haltemittel nach einem der Ansprüche 5 bis 14 umfasst, und ein Versiegelungsmittel zum Versiegeln eines um das Haltemittel gewickelten Folienstreifens für die Herstellung einer länglichen, vorzugsweise im Wesentlichen zylindrischen, Tamponverpackung.

## Revendications

1. Procédé d'enroulement d'une bande de film autour d'un moyen de maintien, comprenant les étapes consistant à :
a. fournir une bande, de préférence une bande quadrilatérale et plus préférablement une bande rectangulaire, de préférence un film thermosoudable ;
b. fournir un moyen de maintien allongé ayant un axe longitudinal parallèle à la bande ;
c. déplacer la bande le long d'un axe de déplacement sensiblement perpendiculaire audit axe longitudinal vers le moyen de maintien et/ou déplacer le moyen de maintien le long d'un axe de déplacement sensiblement perpendiculaire audit axe longitudinal vers la bande ;
d. presser la bande contre le moyen de maintien entre une première extrémité de la bande et une seconde extrémité de la bande ;
e. tandis que la bande est pressée contre le moyen de maintien, enrouler la première extrémité de la bande autour du moyen de maintien dans une première direction de rotation et enrouler la seconde extrémité de la bande autour du moyen de maintien dans une seconde direction de rotation opposée à la première direction de rotation ;
f. immobiliser, facultativement, la bande de film enroulée par rapport au moyen de maintien ; et
g. sceller, facultativement, de préférence thermosouder, un chevauchement de la première et de la seconde extrémité de la bande.

2. Procédé d'enroulement d'une bande de film autour d'un moyen de maintien, selon la revendication précédente 1, moyennant quoi l'enroulement de la première extrémité de la bande autour du moyen de maintien et l'enroulement de la seconde extrémité de la bande autour du moyen de maintien sont exécutés de manière à ce qu'au moins une partie de la première extrémité chevauche au moins une partie de la seconde extrémité de la bande, **caractérisé en ce que** ladite au moins une partie de la seconde extrémité de la bande est enroulée entre le moyen de maintien et ladite au moins une partie de la première extrémité de la bande.

3. Procédé d'enroulement d'une bande de film autour d'un moyen de maintien, selon l'une quelconque des revendications précédentes 1 ou 2, moyennant quoi la bande est pressée contre le moyen de maintien entre une première extrémité de la bande et une seconde extrémité de la bande dans une première zone de pressage et une seconde zone de pressage, **caractérisé en ce que** la première zone de pressage se déplace de façon continue sur le moyen de maintien pendant l'enroulement de la première extrémité de la bande et la seconde zone de pressage se déplace de façon continue sur le moyen de maintien pendant l'enroulement de la seconde extrémité de la bande.

4. Procédé d'enroulement d'une bande de film autour d'un moyen de maintien, selon l'une quelconque des revendications précédentes 1 à 3, **caractérisé en ce qu'**à l'étape c, la bande est déplacée le long de l'axe de déplacement sensiblement perpendiculairement à l'axe longitudinal vers le moyen de maintien, de préférence dans lequel le moyen de maintien reste immobile pendant l'étape de déplacement de la bande, de pressage de la bande et d'enroulement de la bande, et de préférence pendant l'étape de scellement de la bande, et/ou dans lequel, dans l'étape c, le moyen de maintien est déplacé le long d'un axe de déplacement sensiblement perpendiculairement audit axe longitudinal vers la bande, de préférence dans lequel le boîtier reste immobile pendant l'étape de déplacement de la bande, de pressage de la bande et d'enroulement de la bande, et de préférence pendant l'étape de scellement de la bande.

5. Appareil pour enrouler une bande de film autour d'un moyen de maintien allongé pour fabriquer un emballage de tampon hygiénique allongé, de préférence sensiblement cylindrique, comprenant un support et un boîtier, l'appareil comprenant en outre un premier ensemble de moyens d'enserrement (4a) et un second ensemble de moyens d'enserrement (4b), le premier ensemble de moyens d'enserrement étant monté rotatif sur le boîtier (2) autour d'un premier axe de pivotement (8a) et le second ensemble de moyens d'enserrement étant monté rotatif sur le boîtier autour d'un second axe de pivotement (8b) qui est sensiblement parallèle au premier axe de pivotement ;
dans lequel le boîtier (2) est monté de façon déplaçable sur le support (3) et est mobile de façon réversible par rapport audit support le long d'un axe de déplacement sensiblement vers le moyen de maintien (1) pour pousser la bande de film vers le moyen de maintien par au moins desdits moyens d'enserrement (4a, 4b), ledit axe de déplacement étant sensiblement perpendiculaire auxdits premier et second axes de pivotement (8a, 8b), définissant ainsi une direction de pincement sensiblement perpendiculaire audit axe de déplacement et auxdits premier et second axes de pivotement ; et/ou dans lequel le moyen de maintien (1) est mobile de façon réversible le long d'un axe de déplacement sensiblement vers le boîtier (2) pour pousser la bande de film vers les moyens d'enserrement (4a, 4b), ledit axe de déplacement étant sensiblement perpendiculaire auxdits premier et second axes de pivotement (8a, 8b), définissant ainsi une direction de pincement sensiblement perpendiculaire audit axe de déplacement et auxdits premier et second axes de pivotement ;
dans lequel chaque ensemble de moyens d'enserrement (4a, 4b) comprend au moins un moyen d'enserrement (5a, 5a', 5a", 5b, 5b', 5b") comprenant :
- un moyen de pression (6) pour presser la bande de film entre les moyens d'enserrement et le moyen de maintien ; et
- un moyen d'enroulement (6, 7) pour enrouler la bande de film autour du moyen de maintien,
dans lequel le moyen d'enroulement (6a, 6a', 6a", 7a, 7a', 7a") des moyens d'enserrement du premier ensemble de moyens d'enserrement est configuré pour exécuter un mouvement d'enroulement pour enrouler la bande de film autour du moyen de maintien (1) dans une première direction de rotation tandis que les moyens d'enserrement sont déplacés et les moyens de pression sont pressés contre le moyen de maintien, et
dans lequel le moyen d'enroulement (6b, 6b', 6b", 7b, 7b', 7b") des moyens d'enserrement du second ensemble de moyens d'enserrement est configuré pour exécuter un mouvement d'enroulement pour enrouler la bande de film autour du moyen de maintien dans une seconde direction de rotation, opposée à la première direction de rotation, tandis que les moyens d'enserrement sont déplacés et les moyens de pression sont pressés contre le moyen de maintien.

6. Appareil selon la revendication précédente 5, dans lequel le moyen de pression d'au moins un moyen d'enserrement du premier ensemble de moyens d'enserrement peut être déplacé le long au moins de la direction de pincement par rapport au boîtier et moyennant quoi le moyen d'enserrement est configuré de manière à ce qu'un déplacement par rapport au boîtier dudit moyen de pression dudit au moins un moyen d'enserrement du premier ensemble de moyens d'enserrement le long au moins de la direction de pincement actionne le mouvement d'enroulement du moyen d'enroulement dudit au moins un moyen d'enserrement du premier ensemble de moyens d'enserrement, de préférence dans lequel les moyens de pression de tous les moyens d'enserrement du premier ensemble de moyens d'enserrement peuvent être déplacés le long au moins de la direction de pincement par rapport au boîtier, et moyennant quoi tous les moyens d'enserrement sont configurés de manière à ce qu'un déplacement par rapport au boîtier dudit moyen de pression d'un ou plusieurs des moyens d'enserrement du premier ensemble de moyens d'enserrement le long au moins de la direction de pincement actionne le mouvement d'enroulement du moyen d'enroulement desdits un ou plusieurs des moyens d'enserrement du premier ensemble de moyens d'enserrement ;
et/ou dans lequel le moyen de pression d'au moins un moyen d'enserrement du second ensemble de moyens d'enserrement peut être déplacé le long au moins de la direction de pincement par rapport au boîtier, et moyennant quoi le moyen d'enserrement est configuré de manière à ce qu'un déplacement par rapport au boîtier dudit moyen de pression dudit au moins un moyen d'enserrement du second ensemble de moyens d'enserrement le long au moins de la direction de pincement, actionne le mouvement d'enroulement du moyen d'enroulement dudit au moins un moyen d'enserrement du second ensemble de moyens d'enserrement, de préférence dans lequel les moyens de pression de tous les moyens d'enserrement du second ensemble de moyens d'enserrement peuvent être déplacés le long au moins de la direction de pincement par rapport au boîtier, et moyennant quoi tous les moyens d'enserrement sont configurés de manière à ce qu'un déplacement par rapport au boîtier dudit moyen de pression d'un ou plusieurs des moyens d'enserrement du second ensemble de moyens d'enserrement le long au moins de la direction de pincement actionne le mouvement d'enroulement du moyen d'enroulement desdits un ou plusieurs des moyens d'enserrement du second ensemble de moyens d'enserrement.

7. Appareil selon l'une quelconque des revendications précédentes 5 ou 6, dans lequel le moyen de pression d'au moins un moyen d'enserrement du premier ensemble de moyens d'enserrement est conçu pour se déplacer de façon continue sur le moyen de maintien dans la seconde direction de rotation tandis que ledit au moins un moyen d'enserrement du premier ensemble de moyens d'enserrement est déplacé, de préférence dans lequel les moyens de pression de tous les moyens d'enserrement du premier ensemble de moyens d'enserrement sont conçus pour se déplacer de façon continue sur le moyen de maintien dans la seconde direction de rotation tandis que les moyens d'enserrement du premier ensemble de moyens d'enserrement sont déplacés ;
et/ou dans lequel le moyen de pression d'au moins un moyen d'enserrement du second ensemble de moyens d'enserrement est conçu pour se déplacer de façon continue sur le moyen de maintien dans la première direction de rotation tandis que ledit au moins un moyen d'enserrement du second ensemble de moyens d'enserrement est déplacé, de préférence dans lequel les moyens de pression de tous les moyens d'enserrement du second ensemble de moyens d'enserrement sont conçus pour se déplacer de façon continue sur le moyen de maintien dans la première direction de rotation tandis que les moyens d'enserrement du second ensemble de moyens d'enserrement sont déplacés.

8. Appareil selon l'une quelconque des revendications précédentes 5 à 7, moyennant quoi le premier ensemble de moyens d'enserrement est capable d'enrouler une première extrémité de la bande autour du moyen de maintien dans la première direction de rotation, et moyennant quoi le second ensemble de moyens d'enserrement est capable d'enrouler une seconde extrémité de la bande, opposée à la première extrémité de la bande, autour du moyen de maintien dans la seconde direction de rotation, moyennant quoi au moins une partie de la première extrémité enroulée de la bande et au moins une partie de la seconde extrémité enroulée de la bande sont superposées, et est **caractérisé en ce que** le premier ensemble de moyens d'enserrement et le second ensemble de moyens d'enserrement sont configurés l'un par rapport à l'autre de sorte que la partie superposée de la première extrémité de la bande est positionnée entre le moyen de maintien et la partie superposée de la seconde extrémité de la bande.

9. Appareil selon l'une quelconque des revendications précédentes 5 à 8, comprenant :
a. un moyen de fourniture de film pour fournir le film ;
b. un moyen de coupe pour couper le film, formant ainsi la bande de film et fournissant ladite bande de film entre le moyen de maintien et les premier et second ensembles de moyens d'enserrement ; et
c. facultativement, un moyen de support pour supporter la bande de film entre le moyen de maintien et les premier et second ensembles de moyens d'enserrement.

10. Appareil selon l'une quelconque des revendications précédentes 5 à 9, comprenant un moyen de transport qui comprend un ou plusieurs desdits moyens de maintien et est configuré pour fournir les moyens de maintien dans un processus par à-coups dans une position stationnaire qui est sensiblement éloignée du premier et du second ensemble de moyens d'enserrement le long de l'axe de déplacement, de préférence moyennant quoi le moyen de transport est une roue de transport capable de tourner avec une multitude desdits moyens de maintien disposés de façon équidistante le long du bord de ladite roue de transport.

11. Appareil selon l'une quelconque des revendications précédentes 5 à 10, moyennant quoi le moyen de pression d'au moins un moyen d'enserrement du premier et/ou du second ensemble de moyens d'enserrement comprend un ou plusieurs rouleaux, de préférence les moyens de pression de tous les des moyens d'enserrement du premier et du second ensemble de moyens d'enserrement comprennent un ou plusieurs rouleaux, moyennant quoi lesdits un ou plusieurs rouleaux sont conçus pour se déplacer de façon continue sur le moyen de maintien tandis que le boîtier est déplacé le long de l'axe de déplacement et tandis que lesdits un ou plusieurs rouleaux exercent une pression contre le moyen de maintien.

12. Appareil selon l'une quelconque des revendications précédentes 5 à 11, moyennant quoi le moyen d'enroulement d'au moins un moyen d'enserrement du premier et/ou du second ensemble de moyens d'enserrement comprend une ou plusieurs extensions, de préférence moyennant quoi le moyen d'enroulement de tous les moyens d'enserrement du premier et du second ensemble de moyens d'enserrement comprend une ou plusieurs extensions, moyennant quoi lesdites une ou plusieurs extensions sont conçues pour exécuter le mouvement d'enroulement pour enrouler la bande de film autour du moyen de maintien tandis que le boîtier est déplacé le long de l'axe de déplacement et tandis que lesdits un ou plusieurs moyens de pression exercent une pression contre le moyen de maintien.

13. Appareil selon l'une quelconque des revendications précédentes 5 à 12, moyennant quoi au moins un des moyens d'enserrement, de préférence tous, du premier et/ou du second ensemble sont reliés au boîtier par un ou plusieurs ressorts, et moyennant quoi les ressorts sont configurés pour actionner le pressage des moyens de pression contre le moyen de maintien.

14. Appareil selon l'une quelconque des revendications précédentes 5 à 13, dans lequel les moyens d'enroulement sont conçus pour enrouler la bande de film autour du moyen de maintien de telle sorte qu'une première extrémité de la bande et une seconde extrémité opposée de la bande de film sont superposées, et moyennant quoi l'appareil comprend un ou plusieurs moyens de fixation conçus pour serrer les extrémités superposées de la bande de film enroulée et/ou souder, de préférence thermosouder, lesdites extrémités superposées de la bande de film enroulée, de préférence moyennant quoi l'appareil comprend un ou plusieurs desdits moyens de fixation pour chacun des moyens de maintien.

15. Appareil de fabrication d'emballage de tampon hygiénique comprenant un ou plusieurs moyens de maintien, un appareil pour enrouler une bande de film autour d'un moyen de maintien selon l'une quelconque des revendications 5 à 14, et un moyen de scellage pour sceller une bande de film enroulée autour du moyen de maintien pour fabriquer un emballage de tampon allongé, de préférence sensiblement cylindrique.
